(19) Europäisches Patentamt
European Patent Office
Office européen des brevets

(11) Veröffentlichungsnummer: **0 354 508**
**A2**

# (12) EUROPÄISCHE PATENTANMELDUNG

(21) Anmeldenummer: 89114523.7

(22) Anmeldetag: 07.08.89

(51) Int. Cl.⁴: C07D 311/72 , C07D 407/04 , C07D 409/06 , C07D 405/06 , A61K 31/355

Patentansprüche für folgende Vertragsstaaten: ES + GR.

(30) Priorität: 09.08.88 US 230094
16.06.89 US 367082

(43) Veröffentlichungstag der Anmeldung: 14.02.90 Patentblatt 90/07

(84) Benannte Vertragsstaaten: AT BE CH DE ES FR GB GR IT LI LU NL SE

(71) Anmelder: F. HOFFMANN-LA ROCHE AG
Postfach 3255
CH-4002 Basel(CH)

(72) Erfinder: Walser, Armin
19 Crane Avenue
West Caldwell, N.J.07006(US)

(74) Vertreter: Lederer, Franz, Dr. et al
Patentanwalt Dr. Franz Lederer
Lucile-Grahn-Strasse 22
D-8000 München 80(DE)

## (54) Benzopyranolderivate.

(57) Benzopyranolderivate der Formel

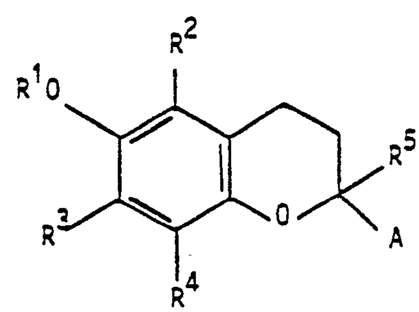

I

worin A -C≡C-$R^6$, -$CH_2CH_2$-$R^7$ oder

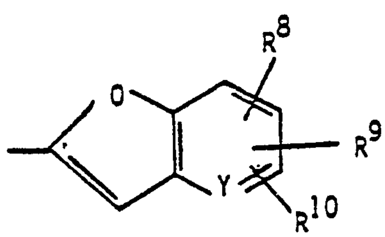

A'

und $R^1$ Wasserstoff oder nieder-Alkanoyl,
$R^2$, $R^3$ und $R^4$ Wasserstoff oder nieder-Alkyl,

EP 0 354 508 A2

$R^5$ nieder-Alkyl,

$R^6$ und $R^7$ ein heteroaromatisches Radikal oder ein aromatisches Radikal aus der Gruppe von Phenyl, Naphthyl oder Phenanthryl, wobei ein solches aromatisches Radikal $R^6$ oder $R^7$ unsubstituiert oder durch einen oder mehrere Reste $Z^6$ bzw. $Z^7$ substituiert sein kann,

$Z^6$ Phenyl-nieder-alkoxy, Hydroxyimino-nieder-alkyl oder eine Gruppe Z,

$Z^7$ Phenyl-$C_{2-7}$-alkoxy oder eine Gruppe Z, Z, Cl, F, nieder-alkyl, nieder-Alkoxy, nieder-Alkanoyl, nieder-Alkanoyloxy, Hydroxy-nieder-alkyl, COOH, nieder-Alkoxycarbonyl, $NH_2$, Amino-nieder-alkyl, Mono- oder Di-nieder-alkylamino, Mono- oder Di-nieder-alkylamino-nieder-alkyl, nieder-Alkanoylamino, $CONH_2$, Mono- oder Di-nieder-alkylcarbamoyl, Trifluoracetylamino, $CF_3$, OH oder Pyridyl ist; oder Z mit zwei benachbarten C-Atomen die Gruppierung $-OCH_2CH_2N(R'')-$ oder $-OCH_2CON(R''')-$ bildet,

$R''$ H, nieder Alkanoyl oder Trifluoracetyl,

$R'''$ H oder nieder Alkyl,

$R^8$, $R^9$ und $R^{10}$ H, OH, nieder Alkyl, nieder Alkoxy, Hydroxy-nieder-alkyl, F, Cl oder nieder-Alkanoyl sind, jedoch nicht mehr als eins von $R^8$, $R^9$ oder $R^{10}$ OH, nieder-Alkoxy, Hydroxy-nieder-alkyl, F, Cl oder nieder-Alkanoyl ist, und

Y CH oder N ist,

und Salze davon.

Die Verbindungen der Formel I sind als Inhibitoren der 5-Lipoxygenase und der Lipidperoxidation wirksam. Sie sind daher bei der Behandlung von Krankheiten, die durch übermässigen oxidativen Metabolismus der Arachidonsäure via 5-Lipoxygenase verursacht sind, sowie bei der Behandlung von Inflammationen, Arthritis, Allergien, Asthma und Psoriasis von Wert. Die Verbindungen der Formel I können auch zur Vorbeugung der Peroxidation der Lipide und daher zum Schutz der Lipidmembranen gegen oxidative Beanspruchung Verwendung finden.

Sie können hergestellt werden ausgehend von Verbindungen der Formel I, worin A für Aethinyl steht.

## Benzopyranolderivate

Die Erfindung betrifft Benzopyranolderivate der Formel

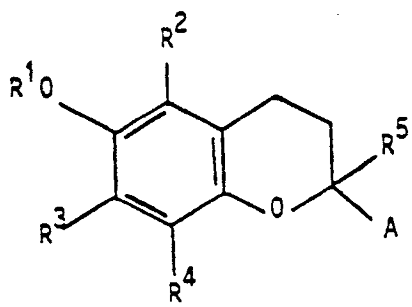

I

worin A $-C{\equiv}C-R^6$, $-CH_2CH_2-R^7$ oder

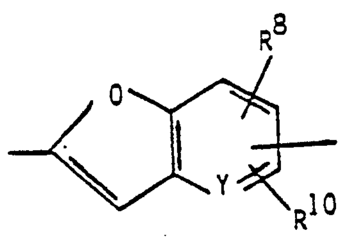

A'

und $R^1$ Wasserstoff oder nieder-Alkanoyl,
$R^2$, $R^3$ und $R^4$ Wasserstoff oder nieder-Alkyl,
$R^5$ nieder-Alkyl,
$R^6$ und $R^7$ ein heteroaromatisches Radikal oder ein aromatisches Radikal aus der Gruppe von Phenyl, Naphthyl oder Phenanthryl, wobei ein solches aromatisches Radikal $R^6$ oder $R^7$ unsubstituiert oder durch einen oder mehrere Reste $Z^6$ bzw. $Z^7$ substituiert sein kann,
$Z^6$ Phenyl-nieder-alkoxy, Hydroxyimino-nieder-alkyl oder eine Gruppe Z,
$Z^7$ Phenyl-$C_{2-7}$-alkoxy oder eine Gruppe Z,
Z Cl, F, nieder-Alkyl, nieder-Alkoxy, nieder-Alkanoyl, nieder-Alkanoyloxy, Hydroxy-nieder-alkyl, COOH, nieder-Alkoxycarbonyl, $NH_2$, Amino-nieder-alkyl, Mono- oder Di-nieder-alkylamino, Mono- oder Di-nieder-alkylamino-nieder-alkyl, nieder-Alkanoylamino, $CONH_2$, Mono- oder Di-nieder-alkylcarbamoyl, Trifluoracetylamino, $CF_3$, OH oder Pyridyl ist; oder Z mit zwei benachbarten C-Atomen die Gruppierung $-OCH_2CH_2N(R'')-$ oder $-OCH_2CON(R''')-$ bildet,
$R''$ H, nieder Alkanoyl oder Trifluoracetyl,
$R'''$ H oder nieder Alkyl,
$R^8$, $R^9$ und $R^{10}$ H, OH, nieder Alkyl, nieder Alkoxy, Hydroxy-nieder-alkyl, F, Cl oder nieder-Alkanoyl sind, jedoch nicht mehr als eins von $R^8$, $R^9$ oder $R^{10}$ OH, nieder-Alkoxy, Hydroxy-nieder-alkyl, F, Cl oder nieder-Alkanoyl ist, und
Y CH oder N ist,
und Salze davon.

Die Verbindungen der Formel enthalten ein asymmetrisches C-Atom und können daher als Enantiomere oder racemische Gemische vorliegen. Die Auftrennung der Racemate in die optisch aktiven Isomeren kann in an sich bekannter Weise durchgeführt werden. So kann man ein racemisches Gemisch eines Vorläufers einer Verbindung der Formel II mit einem optisch aktiven Auftrennungsmittel, z.B. einer optisch aktiven Base, wie R-(+)-α-Methylbenzylamin, umsetzen. Die gebildeten Diastereomere können durch selektive Kristallisation und Ueberführung in die entsprechenden optischen Isomeren abgetrennt werden. Selbstverständlich betrifft die Erfindung sowohl die Racemate der Formel I wie die optisch aktiven Isomere (Enantiomere).

Im Rahmen die Erfindung bezeichnet der Ausdruck "nieder-Alkyl" allein oder in zusammengesetzten Ausdrücken wie in "nieder-Alkoxy" geradkettige oder verzweigte Kohlenwasserstoffreste mit 1-7, vorzugsweise 1-4 C-Atome. Beispiele von solchen Alkylresten sind Methyl, Aethyl, Propyl, Isopropyl, Butyl, t-Butyl,

Neopentyl, Pentyl und Heptyl. Beispiele von nieder Alkoxyresten sind Methoxy, Aethoxy, Propoxy und Pentoxy. "Nieder Alkanoyl" bezeichnet Reste mit 1-7, vorzugsweise 1-4 C-Atomen, wie Formyl, Acetyl, Propanoyl und Butanoyl.

Der Ausdruck "heteroaromatisches Radikal" bezeichnet ein aromatisches monocyclisches 5- oder 6-gliedriges Radikal oder ein bicyclisches Radikal mit einem oder mehreren Heteroatome N, O oder S. Es kann durch eine oder mehrere nieder-Alkyl- oder nieder Alkoxygruppen, Chlor oder Fluor substituiert sein. Beispiele von heteroaromatischen Radikalen sind Pyridyl, Imidazolyl, Thienyl, 2-Chlorthienyl, 2-(Pyridyl)-thienyl, 2-Phenylthienyl, Furyl, Pyrimidinyl, Oxazolyl, Chinolyl, Isochinolyl, Indolyl, Benzothienyl und Benzofuranyl.

Bevorzugte Verbindungen der Formel I sind diejenigen, worin $R^1$ Wasserstoff, $R^2$ bis $R^5$ Methyl und A die Gruppe $A'$ oder vorzugsweise $-C{\equiv}C-R^6$, insbesondere diejenigen, worin $R^6$ unsubstituiertes Phenyl, Thienyl, Benzothienyl oder Pyridyl oder substituiertes Phenyl ist.

Bevorzugt sind auch die Verbindungen der Formel I, worin $R^1$ Wasserstoff, $R^2$ bis $R^5$ Methyl und A die Gruppe $A'$, worin Y Stickstoff und $R^8$ bis $R^{10}$ Wasserstoff oder nieder-Alkyl sind.

Besonders bevorzugt sind folgende Verbindungen:
R-[2-(Benzo[b]thien-3-yl)äthinyl]-3,4-dihydro-2,5,7,8-tetramethyl-2H-1-benzopyran-6-ol;
S-[2-(Benzo[b]thien-3-yl)äthinyl]-3,4-dihydro-2,5,7,8-tetramethyl-2H-1-benzopyran-6-ol;
rac-[2-(Benzo[b]thien-3-yl)äthinyl]-3,4-dihydro-2,5,7,8-tetramethyl-2H-1-benzopyran-6-ol;
R-3,4-Dihydro-2,5,7,8-tetramethyl-2-[(2-thienyl)äthinyl]-2H-1-benzopyran-6-ol;
S-3,4-Dihydro-2,5,7,8-tetramethyl-2-[(2-thienyl)äthinyl]-2H-1-benzopyran-6-ol;
rac-3,4-Dihydro-2,5,7,8-tetramethyl-2-[(2-thienyl)äthinyl]-2H-1-benzopyran-6-ol;
rac-3,4-Dihydro-2-(furo[3,2-b]pyridin-2-yl)-2,5,7,8-tetramethyl-2H-1-benzopyran-6-ol;
rac-2-(2-Benzofuranyl)-3,4-dihydro-2,5,7,8-tetramethyl-2H-1-benzopyran-6-ol; und
rac-3,4-Dihydro-6-hydroxy-2,5,7,8-tetramethyl-2H-1-benzopyran-2-yl)-7-methoxybenzofuran-5-methanol.

Weitere bevorzugte Verbindungen sind folgende:
rac-[2-(Benzo[b]thien-2-yl)äthinyl]-3,4-dihydro-2,5,7,8-tetramethyl-2H-1-benzopyran-6-ol;
rac-3,4-Dihydro-2,5,7,8-tetramethyl-2-[2-(3-pryidinyl)äthinyl]-2H-1-benzopyran-6-ol;
rac-3,4-Dihydro-2,5,7,8-tetramethyl-2-(2-phenyläthyl)-2H-1-benzopyran-6-ol;
rac-3,4-Dihydro-2,5,7,8-tetramethyl-2-(2-phenyläthinyl)-2H-1-benzopyran-6-ol;
rac-3,4-Dihydro-2,5,7,8-tetramethyl-2-[2-(2-thienyl)äthyl]-2H-1-benzopyran-6-ol;
rac-4-[2-(3,4-Dihydro-6-hydroxy-2,5,7,8-tetramethyl-2H-1-benzopyran-2-yl)äthyl]-1,2-benzenediol;
rac-3,4-Dihydro-2,5,7,8-tetramethyl-2-[2-(3-pyridinyl)äthyl]-2H-1-benzopyran-6-ol;
rac-3,4-Dihydro-2,5,7,8-tetramethyl-2-[2-(2-pyridinyl)äthyl]-2H-1-benzopyran-6-ol;
rac-3,4-Dihydro-2-(5-methylfuro[3,2-b]pyridin-2-yl)-2,5,7,8-tetramethyl-2H-1-benzopyran-6-ol; und
rac-2-(3,4-Dihydro-6-hydroxy-2,5,7,8-tetramethyl-2H-1-benzopyran-2-yl)-7-methoxy-5-benzofurancarboxaldehyd.

Weitere Beispiele von Verbindungen der Formel I sind:
rac-3,4-Dihydro-2,5,7,8-tetramethyl-2-(2-hydroxyphenyläthinyl)-2H-1-benzopyran-6-ol;
rac-3,4-Dihydro-2-[(4-hydroxy-3-propylphenyl)äthinyl]2,5,7,8-tetramethyl-2H-1-benzopyran-6-ol;
rac-2-[(3-Acetyloxy-4-methoxyphenyl)äthinyl]-3,4-dihydro-2,5,7,8-tetramethyl-2H-1-benzopyran-6-ol;
rac-5-[(3,4-Dihydro-6-hydroxy-2,5,7,8-tetramethyl-2H-1-benzopyran-2-yl)äthinyl]-2-hydroxybenzoesäure-methylester;
rac-3,4-Dihydro-2,5,7,8-tetramethyl-2-{[5-(2-pyridinyl)-2-thienyl]äthinyl}-2H-1-benzopyran-6-ol;
rac-3,4-Dihydro-2,5,7,8-tetramethyl-2-{[4-(3-pyridinyl)phenyl]äthinyl}-2H-1-benzopyran-6-ol;
rac-3,4-Dihydro-2,5,7,8-tetramethyl-2-[(2-pyridinyl)äthinyl]-2H-1-benzopyran-6-ol;
rac-2-[(5-Butyl-2-thienyl)äthinyl]-3,4-dihydro-2,5,7,8-tetramethyl-2H-1-benzopyran-6-ol;
rac-3,4-Dihydro-2,5,7,8-tetramethyl-2-[(3-chinolinyl)äthinyl]-2H-1-benzopyran-6-ol;
rac-3,4-Dihydro-2,5,7,8-tetramethyl-2-[(1-naphthalinyl)äthinyl]-2H-1-benzopyran-6-ol;
rac-2-{[3-Acetyloxy-4-(phenylmethoxy)phenyl]äthinyl}-3,4-dihydro-2,5,7,8-tetramethyl-2H-1-benzopyran-6-ol;
rac-3,4-Dihydro-2-{[3-hydroxy-4-(phenylmethoxy)phenyl]äthinyl}-2,5,7,8-tetramethyl-2H-1-benzopyran-6-ol;
rac-2-[(3-Acetyloxy-4-methoxyphenyl)äthyl]-3,4-dihydro-2,5,7,8-tetramethyl-2H-1-benzopyran-6-ol;
rac-2-[(3-Acetyloxy-4-methoxyphenyl)äthinyl]-3,4-dihydro-2,5,7,8-tetramethyl-2H-1-benzopyran-6-ol;
rac-3,4-Dihydro-2,5,7,8-tetramethyl-2-{[5-(2-pyridinyl)-2-thienyl]äthyl}-2H-1-benzopyran-6-ol;
rac-3,4-Dihydro-2,5,7,8-tetramethyl-2-{[5-(2-pyridinyl)-2-thienyl]äthinyl}-2H-1-benzopyran-6-ol;
rac-4-[2-(3,4-Dihydro-6-hydroxy-2,5,7,8-tetramethyl-2H-1-benzopyran-2-yl)äthyl]-1,2-benzenediol-2-acetat;
rac-{2-(5-(Aminomethyl)-2-hydroxy-3-methoxyphenyl]äthyl}-3,4-dihydro-2,5,7,8-tetramethyl-2H-1-benzopyran-6-ol hydrochloride;
rac-1-[2-(3,4-Dihydro-6-hydroxy-2,5,7,8-tetramethyl-2H-1-benzopyran-2-yl)-6-hydroxy-7-propylbenzofuran-5-yl]äthanon;
rac-3-[(3,4-Dihydro-6-hydroxy-2,5,7,8-tetramethyl-2H-1-benzopyran-2-yl)äthinyl]-4-hydroxy-5-methoxybenzaldehyd;
rac-3-[(3,4-Dihydro-6-hydroxy-2,5,7,8-tetramethyl-2H-1-benzopyran-2-yl)äthinyl]-4-hydroxy-5-methoxybenzaldehyd-oxim;
rac-3-[(3,4-Dihydro-6-hydroxy-2,5,7,8-tetramethyl-2H-1-benzopyran-2-yl)äthinyl]-4-hydroxy-5-methoxybenzolmethanol;
rac-2-[(2-Aminophenyl)äthinyl]-3,4-dihydro-2,5,7,8-tetramethyl-2H-1-benzopyran-6-ol;
rac-2,2,2-Trifluor-N-{2-[(3,4-dihydro-2,5,7,8-tetramethyl-2H-1-benzopyran-2-yl)äthinyl]-phenyl}acetamid;
rac-3,4-Dihydro-2-[(3-methoxyphenyl)äthinyl]-2,5,7,8-tetramethyl-2H-1-benzopyran-6-ol;
rac-3,4-Dihydro-2-[(3-methoxyphenyl)äthinyl]-2,5,7,8-tetramethyl-2H-1-benzopyran-6-ol;
rac-2-{[3-(Trifluormethyl)phenyl]äthinyl}-3,4-dihydro-2,5,7,8-tetramethyl-2H-1-benzo-pyran-6-ol;
rac-2-[(3,4-Difluorphenyl)äthinyl]-3,4-dihydro-2,5,7,8-tetramethyl-2H-1-benzopyran-6-ol;
rac-3,4-Dihydro-2-[(4-isochinolinyl)äthinyl]-2,5,7,8-tetramethyl-2H-1-benzopyran-6-ol;
rac-2-[(3,4-Dihydro-2H-1,4-benzoxazin-6-yl)äthinyl]-3,4-dihydro-2,5,7,8-tetramethyl-2H-1-benzopyran-6-ol; hydrochlorid-hydrat;
rac-[(3,4-Dihydro-6-hydroxy-2,5,7,8-tetramethyl-2H-1-benzopyran-2-yl)äthinyl]-4-methyl-2H-1,4-benzoxazin-3(4H)-on;
rac-3,4-Dihydro-2-[2-(3-methoxyphenyl)äthyl]-2,5,7,8-tetramethyl-2H-1-benzopyran-6-ol;
rac-2-[2-(5-Butyl-2-thienyl)äthyl]-3,4-dihydro-2,5,7,8-tetramethyl-2H-1-benzopyran-6-ol; und
rac-5-[2-(3,4-Dihydro-6-hydroxy-2,5,7,8-tetramethyl-2H-1-benzopyran-2-yl)äthyl]-2-hydroxybenzoesäure-methylester.

Die Verbindungen der Formel I kann man dadurch herstellen, dass man

a) eine Verbindung der Formel

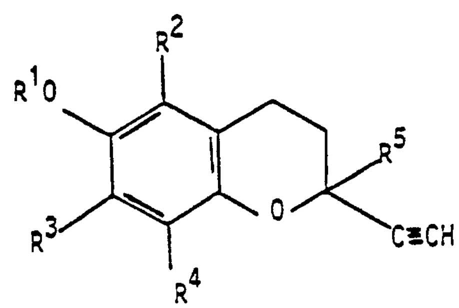

II

mit einer Verbindung der Formel $R^6X$, worin X Br, I oder Trifluormethylsulfonyloxy sind und $R^1$-$R^6$ die obige Bedeutung haben, umsetzt oder

b) eine Verbindung der Formel I, worin A -C≡C-$R^6$ ist, hydriert,

c) eine durch die Reaktion a) entstandene Verbindung der Formel I, worin A -C≡C-$R^{61}$ und $R^{61}$ die Gruppe der Formel

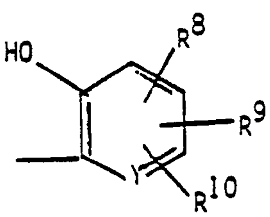

$R^{61}$

ist, in dieser form oder in Form der entsprechenden Verbindung der Formel I, worin A die Gruppe A′ ist, isoliert,

d) gewünschtenfalls eine nach Reaktion a), b) oder c) entstandene Verbindung der Formel I in ein Salz überführt.

Die obige Reaktion a) kann man in Gegenwart eines Palladiumkatalysators wie Bis(triphenylphosphin)-palladium-dichlorid oder -diacetat und in Gegenwart eines Ueberschusses eines Protonenakzeptors, wie Triäthylamin, und gewünschtenfalls in einem inerten organischen Lösungsmittel, wie Acetonitril, Tetrahydrofuran (THF) oder Dimethylformamid, bei einer Temperatur im Bereich von Raumtemperatur bis 100 °C,

durchführen. Die entstandene Verbindung der Formel I, worin A $-C{\equiv}C-R^6$ ist, kann Kristallisation, Destillation oder Chromatographie isoliert werden.

Die Hydrierung b) lässt sich mittels eines Edelmetallkatalysators wie Palladium, Platin oder Nickel, zweckmässig in Gegenwart eines inerten Lösungsmittels, z.B. eines Alkanols wie Aethanol, eines Aethers, wie THF, oder in Essigsäure bei einer Temperatur im Bereich von Raumtemperatur bis 50 °C und unter einem Druck von 1-20 Atm, vorzugsweise unter normalem Druck bewerkstelligen. Die entstandene Verbindung der Formel I, worin A $-CH_2CH_2-R^7$ ist, kann durch Kristallisation oder Chromatographie isoliert werden.

Bei der Reaktion b) entsteht ein Zwischenprodukt der Formel I, worin A für $-CH{\equiv}CH-R^7$ steht. Dieses kann auch mittels eines Lindlar-Katalysators oder mittels Palladium auf Kohlenstoff (Pd/C) in Gegenwart eines Katalysatorengifts wie Thiophen gefolgt von Chromatographie oder Kristallisation erhalten werden.

Eine durch Reaktion a) erhaltene Verbindung der Formel I, worin A eine Gruppe $-C{\equiv}C-R^{61}$ ist und $R^{61}$ obige Bedeutung hat. kann in dieser Form aus dem Reaktionsgemisch isoliert werden. Alternativ kann durch intramolekulare Reaktion der Hydroxygruppe mit der Dreifachbindung eine Verbindung der Formel I, worin A die entsprechende Gruppe A′ ist, entstehen. In beiden Fällen kann die entstandene Verbindung der Formel I z.B. durch Kristallisation oder Chromatographie isoliert werden.

Die Ausgangsmaterialien der Formel II können wie in den nachstehenden Reaktionsschemata 1 und 2 beschrieben hergestellt werden.

Die Verbindungen der Formel IV sind bekannt oder können in an sich bekannter Weise hergestellt werden. Im Reaktionsschema 1 wird eine Verbindung der Formel IV mit Lithiumacetylid in eine Verbindung der Formel V übergeführt. Die Verbindung der Formel V wird mittels Phthalsäureanhydrid in Gegenwart eines tertiären Amins in die entsprechende Verbindung der Formel VI umgewandelt. Ein Hemiphthalat der Formel VI kann mit einem optisch aktiven Amin wie α-Methylbenzylamin umgesetzt werden. Das diastereomere Gemisch von auf diese Weise gebildeten Salze kann durch Kristallisation aufgetrennt werden. Durch Behandlung mit einer wässrigen Säure können die so aufgetrennten Salze in die freien Säuren der Formeln VIa und VIb übergeführt werden.

Reaktionsschema 1

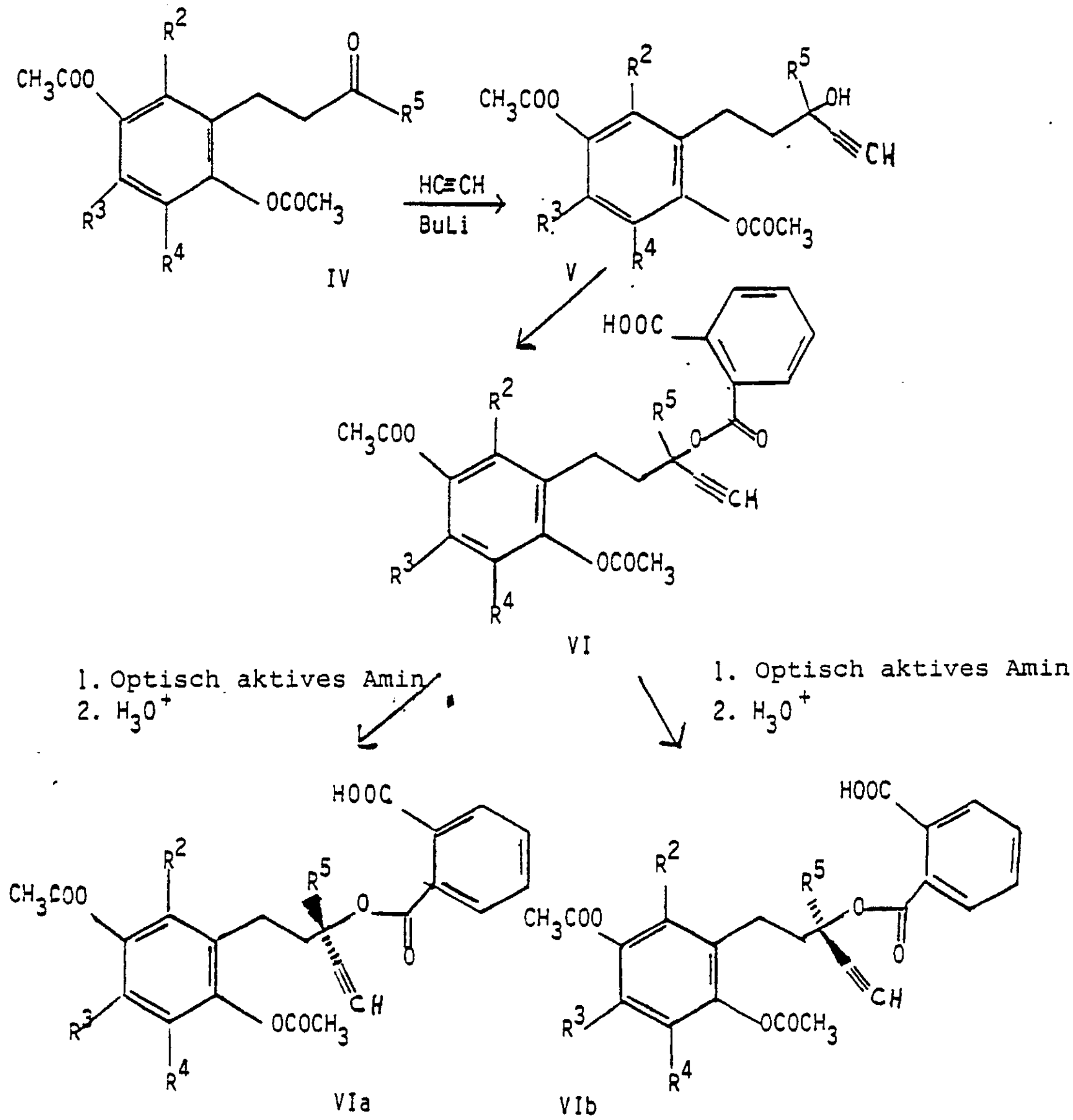


IV V VI VIa VIb

worin $R^2$, $R^3$, $R^4$ und $R^5$ die obige Bedeutung haben.

Nach dem Reaktionsschema 2 können die Verbindungen der Formeln VIa und VIb durch Hydrolyse mit wässrigem Natriumhydroxid in die Verbindungen der Formeln IIa' und IIb' übergeführt werden. Intermediär gebildete Alkohole werden vorzugsweise nicht isoliert, sondern durch Behandlung mit einer Säure in Gegenwart von dreiwertigen Eisenionen in die Verbindungen der Formeln IIa' und IIb' cyclisiert.

Die Verbindungen der Formeln IIa' und IIb' können durch Reaktion mit einem nieder Alkanoylhalogenid der Formel $R''Z$ oder einem Anhydrid der Formel $(R'')_2O$ in die Verbindungen der Formeln IIa'' und IIb'' übergeführt werden. Diese Reaktion kann man in einem inerten organischen Lösungsmittel wie THF oder Methylenchlorid in Gegenwart eines Säureakzeptors wie Pyridin, 4-Dimethylaminopyridin oder Triäthylamin durchführen.

Die Enantiomeren IIa', IIb', IIa'' und IIb'' können mit einer Verbindung der Formel $R^6X$ in die entsprechenden Enantiomeren der Formel I umgewandelt werden.

Reaktionsschema 2

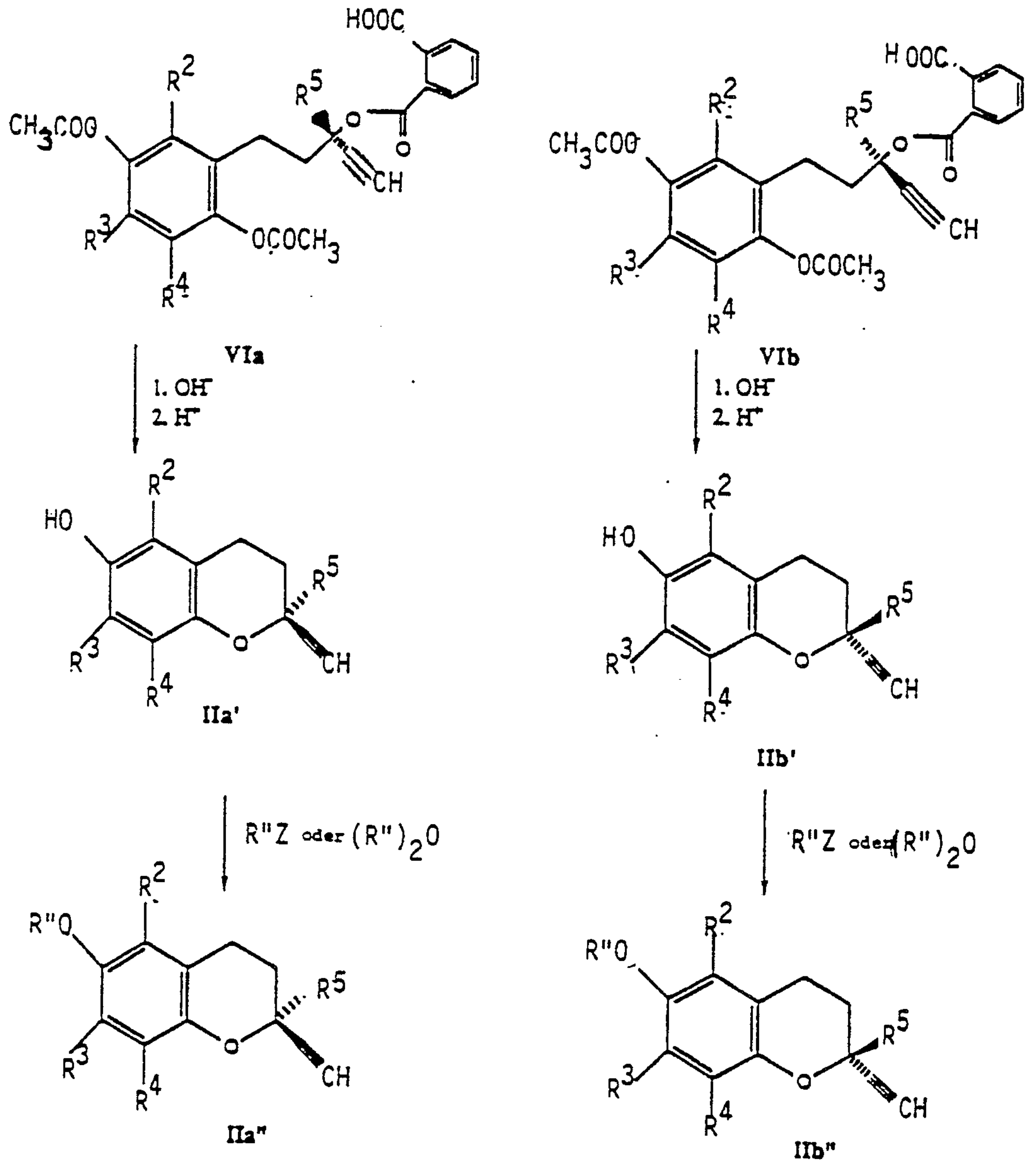


VIa VIb IIa' IIb' IIa" IIb"

worin R" nieder Alkanoyl, Z Halogen und $R^2$, $R^3$, $R^4$ und $R^5$ die obige Bedeutung haben.

Salze der eine Carboxygruppe enthaltenden Verbindungen der Formel I können durch Reaktion mit einer pharmakologisch verwendbaren Base hergestellt werden. Beispiele von solchen Basen sind Alkali- oder Erdalkalimetallhydroxide oder -carbonate, z.B. Calcium- oder Natriumhydroxid, Natrium-oder Kaliumcarbonat, Ammoniak, Amine, wie Mono-, Di- oder Trialkylamine, z.B. Methylamin, Diäthylamin oder Triäthylamin, heterocyclische Amine, wie Piperidin.

Salze von Verbindungen der Formel I mit einer basischen Funktion können hergestellt werden durch Reaktion mit einer pharmazeutisch verwendbaren organischen oder anorganischen Säure, wie Essigsäure, Bernsteinsäure, Ameisensäure, Methansulfonsäure, p-Toluolsulfonsäure, Salzsäure, Salpetersäure, Phosphorsäure oder Schwefelsäure.

Die Verbindungen der Formel I sind als Inhibitoren der 5-Lipoxygenase und der Lipidperoxidation wirksam. Sie sind daher bei der Behandlung von Krankheiten, die durch übermässigen oxidativen Metabolismus der Arachidonsäure via 5-Lipoxygenase verursacht sind, sowie bei der Behandlung von Entzündungen, Arthritis, Allergien, Asthma und Psoriasis von Wert. Die Verbindungen der Formel I können auch zur

Vorbeugung der Peroxidation der Lipide und daher zum Schutz der Lipidmembranen gegen oxidative Beanspruchung Verwendung finden.

## In vitro Test für $\Delta^5$-Lipoxygenasehemmer

Verbindungen der Formel I wurden auf ihre Wirksamkeit auf die $\Delta^5$-Lipoxygenase von RBL-1 Zellen geprüft. Ein Lipoxygenase (5-LO)-Präparat wurde durch Aufbrechen der Zellen mittels eines Homogenisators und Abzentrifugieren der Zelltrümmer gewonnen. Das Enzympräparat wurde mit der Testverbindung oder dem Vehikel 10 Minuten bei 30° C inkubiert, danach wurden die Proben bei 37° C mit Arachidonsäure versetzt, um die 5-LO-Aktivität in Gang zu setzen. Die Werte der durch Enzymreaktion gebildeten Menge von 5-HETE wurde durch einen Radioimmunoassay gemessen. In der Tabelle I sind als $IC_{50}$-Werte diejenigen Konzentrationen der Testverbindung angegeben, die zu einer 50%igen Hemmung der Bildung von 5-HETE im Vergleich zu den Kontrollwerten führte.

## Oedemtest am Mäuseohr in vivo

Die Verbindungen wurden weiterhin im Oedemtest am Mäuseohr getestet. Dabei wird durch Verabreichung von Arachidonsäure in das Ohr ein Oedem induziert; siehe hierzu J. Invest. Dermatol. 80:48 (1983) und Advances in Inflammation Research, 11:57 (1986). Der Versuch wurde so geführt, dass eine Kontrollgruppe weder Arachidonsäure noch Testverbindungen erhielt, eine zweite Gruppe nur Arachidonsäure enthielt und eine dritte Gruppe zunächst die Testverbindungen und danach Arachidonsäure erhielt. Die Testverbindungen wurden, in Aceton gelöst, auf die dorsale Oberfläche des Ohrs aufgebracht. Die Arachidonsäure wurde in der gleichen Weise topisch verabreicht. Die Oedembildung wurde durch Auswägen von Gewebeproben ermittelt. Die prozentuale Hemmung der Oedembildung wurde nach der folgenden Formel berechnet:

$$\frac{\text{Probengewicht der Arachidonsäuregruppe} - \text{Probengewicht der Testgruppe}}{\text{Probengewicht der Arachidonsäuregruppe} - \text{Probengewicht der Kontrollgruppe}} \times 100$$

Die Versuchsdaten sind in Tabelle I angegeben.

## Antiperoxidative Wirkung in vitro

Diese Versuchsanordnung beruht auf der Anwendung von Hypoxanthin-Xanthin-Oxidase als Generator von freiem Radikal und gereinigtem nativem Rattenherz-Membran-Phosphoglycerid als Substrat. Gemessen wird die durch Lipidperoxidation gebildete Menge von Malondialdehyd. Die Versuchsresultate sind in Tabelle III als $IC_{50}$ angegeben, die wie folgt berechnet wurde:

$$\text{\% Hemmung der Peroxidation} = 1 - \frac{\text{Drug}_{60'} - \text{Drug}_{0'}}{T_{60'} - T_{0'}} \times 100$$

$\text{Drug}_{60'}$ = Aequivalente Malondialdehyd, die nach 60 Minuten in Gegenwart des freien Radikalbildners und der Testsubstanz gebildet wurden.
$\text{Drug}_{0'}$ = Aequivalente Malondialdehyd, die nach 0 Minuten in Gegenwart des freien Radikalbilders und der Testsubstanz vorhanden waren.
$T_{60'}$ = Aequivalente Malondialdehyd, die in Abwesenheit der Testverbindungen nach 60 Minuten gebildet wurden.

$T_0'$ = Endogene Thiobarbitursäure-Reaktivität des Reaktionsgemisches zum Zeitpunkt null.

Tabelle I

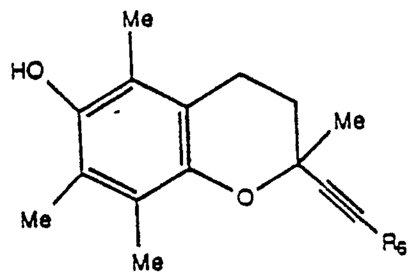

Ia'

| $R^6$ | Beispiel | Hemmung der Lipidperoxidation IC-50 (μM) | Hemmung der 5-LO IC-50 (nM) | Hemmung des Oedems mit 1 mg topisch |
|---|---|---|---|---|
| Phenyl | 1 | 0.65 | 40 | 45 |
| 2-HO-phenyl | | 50 | 0.7 | 6 |
| 2-HO-3-MeO-5-CHO-phenyl | 28 | > 1 | 130 | |
| 2-HO-3-MeO-5-CH2OH-phenyl | 29 | > 1 | 130 | |
| 2-HO-3-MeO-5-CH=NOH-phenyl | | 30 > 1 | 160 | |
| 2-$H_2N$-phenyl | 40 | > 1 | 20 | |
| 2-$F_3CCONH$-phenyl | 44 | | 10 | |
| 3-MeO-phenyl | 22 | > 1 | 170 | |
| 3-$F_3C$-phenyl | 35 | 0.8 | 190 | 15 |
| 3-AcO-4-MeO-phenyl | 20 | > 1 | 24 | |
| 3-AcO-4-$PhCH_2O$-phenyl | 41 | 0.48 | 32 | 31 |
| 3-HO-4-$PhCH_2O$-phenyl | 43 | 0.37 | 24 | 31 |
| 3,4-$F_2$-phenyl | 36 | > 1 | 120 | |
| 3-MeOOC-4-HO-phenyl | 23 | 0.9 | 160 | |
| 4-(3-Pyridyl)phenyl | 24 | 0.8 | 160 | |
| 2-Thienyl, RS | 4 | 0.9 | 23 | 61 |
| 2-Thienyl, R | 5 | 0.8 | 15 | 66 |
| 2-Thienyl, S | 13 | >1 | 17 | 32 |
| 5-(2-Pyridyl)-2-thienyl | 26 | 0.55 | 160 | |
| 3-Benzo(b)thienyl, RS | 16 | 0.56 | 1.6 | 62 |

| $R^6$ | Beispiel | Hemmung der Lipidperoxidation IC-50 (µM) | Hemmung der 5-LO IC-50 (nM) | Hemmung des Oedems mit 1 mg topisch |
|---|---|---|---|---|
| 3-Benzo(b)thienyl, R | 17 | | | |
| 3-Benzo(b)thienyl, S | 18 | | | |
| 2-Benzo(b)thienyl | 19 | | | |
| 2-Pyridyl | 15 | >1 | 16 | 18 |
| 3-Pyridyl | 14 | 0.6 | 40 | 20 |
| 3-Chinolinyl | 33 | 0.85 | 160 | |
| 4-Isochinolinyl | 34 | > 1 | 1000 | |
| 2-(5-Butyl)thienyl | 31 | 0.55 | 1000 | 24 |
| 4-HO-3-propyl-phenyl | 37 | > 1 | | |
| 1-Naphthyl | 39 | > 1 | 500 | |
| 3,4-Dihydro-4-trifluoracetyl-2H-1,4-benzoxazin-6-yl | 45 | | 45 | |
| 3,4-Dihydro-2H-1,4-benzoxazin-6-yl, HCl | 47 | | 18 | |
| 4-Methyl-2H-1,4-benzoxazin-3(4H)-one-6-yl | 48 | 0.75 | 32 | |

Tabelle I (Forts.)

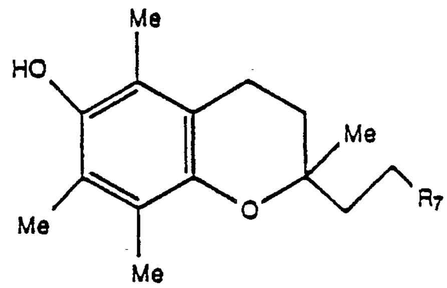

Ib'

| $R^7$ | Beispiel | Hemmung der Lipidperoxidation IC-50 (µM) | Hemmung der 5-LO IC-50 (nM) | Hemmung des Oedems mit 1 mg topisch |
|---|---|---|---|---|
| Phenyl | 56 | > 1 | 110 | 64 |
| 2-Thienyl | 57 | > 1 | 50 | 41 |
| 2-Pyridyl | 58 | 0.7 | 60 | 31 |
| 3-Pyridyl | 59 | 0.5 | 30 | 48 |
| 3-AcO-4-MeO-phenyl | 60 | > 1 | 56 | |
| 3,4-Dihydroxyphenyl | 61 | > 1 | 15 | 55 |
| 3-MeO-phenyl | 62 | > 1 | 120 | |
| 3-MeOOC-4-HO-phenyl | 63 | > 1 | 80 | |
| 4-(3-Pyridyl)-phenyl | 64 | > 1 | 46 | |
| 5-(2-Pyridyl)-2-thienyl | 65 | > 1 | 170 | |
| 5-Butyl-2-thienyl | 66 | > 1 | 140 | |
| 3-AcO-4-HO-phenyl | 67 | | 38 | |
| 2-HO-3-MeO-5-$CH_2NH_2$-phenyl, HCl | 68 | 1 | 70 | |

Tabelle I (Forts.)

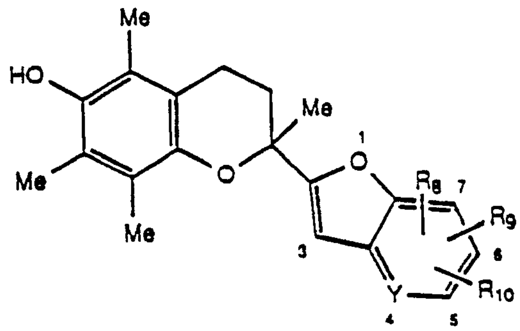

Id'

| Y | $R^8$, $R^9$, $R^{10}$ | Beispiel | Hemmung der Lipidperoxidation IC-50 (µM) | Hemmung der 5-LO IC-50 (nM) | Hemmung des Oedems mit 1 mg topisch |
|---|---|---|---|---|---|
| N | H, H, H | 52 | 0.95 | 18 | 49 |
| N | 5-Methyl, H, H | 51 | > 1 | 3 | 69 |
| CH | H, H, H | 50 | 1.0 | 12 | 68 |
| CH | 5-CHO, 7-MeO, H | 54 | | 1 | 40 |
| CH | 5-$HOCH_2$, 7-MeO, H | 55 | 0.85 | 3 | |
| CH | 5-Acetyl, 6-HO, 7-propyl | 53 | 0.6 | 17 | 54 |

Die Verbindungen der Formel I oder deren Salze können in an sich bekannter Weise verabreicht werden. Die Verbindungen der Formel I können einzeln oder mit anderen Pharmazeutika oral, parenteral, rektal oder durch Inhalierung, beispielsweise in Form eines Aerosols oder eines mikronisierten Pulvers verabreicht werden. Die orale Verabreichung kann in Form von Tabletten, Kapseln, beispielsweise im Gemisch mit Talk, Stärke, Milchzucker oder anderen inerten Ingredienzien, d.h. pharmazeutisch akzeptablen Trägen erfolgen, oder in Form von wässrigen Lösungen, Suspensionen oder wässrig-alkoholischen Lösungen, beispielsweise im Gemisch mit Zucker oder Süsstoffen, Geschmackstoffen, Farbstoffen, Verdickungsmitteln und anderen konventionellen pharmazeutischen Excipientien oder in Form von Kügelchen für die orale Verabreichung. Für die parenterale Verabreichung können die Verbindungen als Lösungen oder Suspensionen, beispielsweise als wässrige Lösung oder in Lösung oder Suspension mit Erdnussöl unter Verwendung von Excipientien und üblichen Trägern angewandt werden. Für die Verabreichung als Aerosole können sie in einem geeigneten pharmazeutisch anwendbaren Lösungsmittel, beispielsweise Aethylalkohol oder Kombinationen mischbarer Lösungsmittel und im Gemisch mit einem pharmazeutisch akzeptablen Treibmittel angewandt werden. Solche Aerosole können in einem Druckbehälter mit Zerstäuberventil abgepackt werden. Für die rektale Verabreichung können die Verbindungen als Suppositorium unter Verwendung eines inerten Trägers wie Kabaobutter eingesetzt werden. Für die topisch Verabreichung können die Verbindungen der Formel I in Salben, Crèmes, Lotionen und Gele eingearbeitet werden. Lösungen, Salben und Crèmes enthalten im allgemeinen absorbierbare wasserlösliche oder emulsionsartige Grundlagen wie Petrolatum, Lanolin und Polyäthylenglykol. Lösungen können die Verbindungen der Formel I in einem pharmazeutisch akzeptablen Lösungsmittel wie Polyäthylenglykol enthalten. Geeignete Lotionen sind echte Lösungen, wässrige oder hydroalkoholische Formulierungen, die fein verteilte Partikel enthalten. Lotionen können Suspendierungs- oder Dispergiermittel wie Cellulosederivate, beispielsweise Methylcellulose und Aethylcellulose enthalten. Gele sind halbfeste Präparate, die aus einer Lösung oder Suspension einer Verbindung der Formel I in einem geeigneten wässrigen oder wasserfreien Vehikel unter Verwendung eines Geliermittels wie Carboxypolymethylen erhalten werden können und die danach durch

Zusatz von Alkalimetallhydroxiden wie Natriumhydroxiden und Aminen, beispielsweise Polyäthylenkokosamin auf die geeignete Konsistenz gebracht werden. Pharmazeutische Präparate zur topischen Verabreichung einer Verbindung der Formel I können auch konventionelle Inhaltsstoffe wie Konservierungsmittel, Stabilisatoren, Befeuchtungsmittel, Emulgatoren und Puffer in üblichen Mengen enthalten, die den jeweiligen Erfordernissen angepasst sind und durch den Fachmann ohne weiteres ermittelt werden können.

Für die Verabreichung der Verbindung der Formel I in oralen Dosen kommen Dosierungen von 25-1000 mg pro Tag in Betracht, vorzugsweise 25-250 mg entweder als Einzeldosis oder in unterteilten Dosen.

Die nachfolgenden Beispiele erläutern die Erfindung weiter.

## Beispiel 1

Ein Gemisch von 2,3 g rac-3,4-Dihydro-2-äthinyl-2,5,7,8-tetramethyl-2H -1-benzopyran-6-ol, 3,06 g Jodbenzol, 0,39 g Triphenylphosphin, 95 mg Kupfer(I)jodid, 3 ml Triäthylamin und 100 ml Acetonitril wurde 10 Minuten mit Argon entgast. 110 mg Palladiumacetat wurden zugesetzt und das Rühren wurde 3 Stunden unter Argon fortgeführt. Das Reaktionsgemisch wurde unter vermindertem Druck eingedampft und der Rückstand wurde zwischen Methylenchlorid und gesättigter Natriumbicarbonatlösung verteilt. Die organische Phase wurde abgetrennt, über Natriumsulfat getrocknet und eingedampft. Das Produkt wurde über Silicagel mittels Methylenchlorid chromatographiert. Die vereinigten reinen Fraktionen wurden eingedampft und der Rückstand wurde aus Hexan kristallisiert. Man erhielt 2,1 g (68 %) rac-3,4-Dihydro-2,5,7,8-tetramethyl-2-(2-phenyläthinyl)-2H -1-benzopyran-6-ol, Smp. 109-111°.

Das Ausgangsmaterial wurde wie in Beispiel 2 beschrieben hergestellt.

## Beispiel 2

rac-3,4-Dihydro-2-äthinyl-2,5,7,8-tetramethyl-2H-1-benzopyran-6-ol.

Eine Lösung von 50 g rac-5-[2,5-Bis(acetyloxy)-3,4,6-trimethylphenyl]-3-methyl -1-pentyn-3-ol [Helv. Chimica Acta, 67, 650 (1963)] in 250 ml Methanol wurde mit Argon entgast. Eine Lösung von 25 g Natriumhydroxid in 30 ml Wasser wurde zugesetzt und das Gemisch 1,5 Stunden unter Argon unter Rückfluss erhitzt. Das Reaktionsgemisch wurde abgekühlt und ein Gemisch von 25 ml konzentrierter Schwefelsäure in 125 ml Methanol wurde zugesetzt. Nach Zusatz von 0,5 g Eisen(III)chlorid-trihydrat wurde des Gemisch 18 Stunden unter Rückfluss erhitzt. Es wurde dann zwischen Methylenchlorid und wässrigem Natriumcarbonat aufgeteilt. Die organische Schicht wurde abgetrennt, getrocknet und teilweise eingedampft. Hexan wurde zugesetzt und die Lösung wurde eingeengt und abgekühlt. Man erhielt 31 g (89%) Kristalle, Smp. 112-114°.

Die Herstellung des Ausgangsmaterials wird in Beispiel 3 beschrieben.

## Beispiel 3

Rac-5-[2,5-Bis(acetyloxy)-3,4,6-trimethylphenyl]-3-methyl-1-pentin-3-ol.

350 ml THF wurden bei -78° mit 17,6 g Acetylen begast. Dieser Lösung wurde unter Argon eine Lösung von 387 ml n-Butyllithium (1,6M in Hexan) so zugetropft, dass die Temperatur zwischen -80 und -65° blieb. Bei -70° wurde dann die Lösung 64,33 g 4-(2,5-Diacetyloxy-3,4,6-trimethylphenylbutan-2-on in 300 ml THF zugetropft. Das Gemisch wurde 1 Stunde bei -70° gerührt, wonach das Abkühlen aufgehört wurde. Als die Temperatur des Reaktionsgemisches -30° erreichte, wurden 500 ml Wasser und dann 50 g Ammoniumchlorid zugesetzt. Das Gemisch wurde mit Stickstoff entgast und auf 25° erwärmen gelassen. Nach 30 Minuten wurde das Gemisch mit Aether extrahiert. Die organischen Extrakte wurden getrocknet, filtriert und eingeengt. Nach Zusatz von Hexan wurde die Lösung beimpft und 18 Stunden bei 4° belassen. Der entstandene Feststoff wurde mit Aether gewaschen und getrocknet. Man erhielt 54,9 g Produkt, Smp.

116-118°. Durch Einengen der Mutterlauge und Kristallisation aus $CH_2Cl_2$/Aether/Hexan erhielt man weitere 13,3 g Produkt.

## Beispiel 4

In zu Beispiel 1 analoger Weise erhielt man aus einem Gemisch von 4,6 g rac-3,4-Dihydro-2-äthinyl-2,5,7,8-tetramethyl-2H -1-benzopyran-6-ol, 6,3 g 2-Jodthiophen, 0,8 g Triphenylphosphin, 0,19 g Kupfer(I)-jodid, 6 ml Triäthylamin und 200 ml Acetonitril, 3,5 g (56%) rac-3,4-Dihydro-2,5,7,8-tetramethyl-2-[(2-thienyl)äthinyl] -2H-1-benzopyran-6-ol, Smp. 112-114° C nach Kristallisation aus Aether/Hexan.

## Beispiel 5

(R)-3,4-Dihydro-2,5,7,8-tetramethyl-2-[(2-thienyl)äthinyl]-2H-1-benzopyran-6-ol.

Diese Verbindung, Smp. 133-135°, $[\alpha]_D^{25}$ = -36,8° (c = 1,056 in MeOH), wurde hergestellt wie in Beispiel 1 beschrieben ausgehend von (R)-3,4-Dihydro-2-äthinyl-2,5,7,8-tetramethyl-2H -1-benzopyran-6-ol.

Das Ausgangsmaterial wurde hergestellt wie beschrieben in den Beispielen 6 bis 12.

## Beispiel 6

Ein Gemisch von 21,25 g rac-5-[2,5-Bis(acetyloxy)-3,4,6-trimethylphenyl]-3-methyl -1-pentyl-3-ol, 12,12 g Triäthylamin, 2,44 g 4-Dimethylaminopyridin und 11,84 g Phthalsäureanhydrid in 100 ml Dichlormethan wurde 3 Stunden bei 25° gerührt und dann 10 Stunden unter Rückfluss erhitzt. Das Reaktionsgemisch wurde auf 25° abgekühlt und mit Diäthyläther verdünnt. Die Lösung wurde mit 1,0 N HCl gewaschen. Die wässrige Phase wurde mit Diäthyläther extrahiert. Die organischen Phasen wurden mit 1,0 N Ammoniumhydroxid extrahiert. Die basischen wässrigen Extrakte wurden abgekühlt und mit 6,0 N HCl auf pH 5,0 angesäuert. Dann wurde mit Dichlormethan extrahiert. Die vereinigten Extrakte wurden getrocknet. Filtrierung und Abdampfen des Lösungsmittels ergab ein Oel. Dieses wurde zu Diäthyläther zugesetzt und das Lösungsmittel wurde abgedampft. Der entstandene Schaum wurde getrocknet und man erhielt 31 g rac-[3-[2,5-Bis(acetyloxy)-3,4,6-trimethylphenyl] -1-äthinyl-1-methylpropyl]-1,2-benzoldicarbonsäureester.

## Beispiel 7

Einer Lösung von 27,00 g des Produktes von Beispiel 6 in 50 ml 95% Aethanol wurden 7,02 g (S)-(-)-α-Methylbenzylamin in 250 ml Diäthyläther zugesetzt. Die entstandene Lösung wurde bei 25° gerührt und dann abgekühlt. Nach Rühren bei 0° wurden die Kristalle gesammelt und zu 16 g Feststoff getrocknet. Dieser wurde in 50 ml Aethanol und 150 ml Diäthyläther gelöst. Die Lösung wurde bei 25° und dann bei 0° gehalten. Nach Trocknen erhielt man 10,1 g (S)-[3-[2,5-Bis(acetyloxy)-3,4,6-trimethylphenyl]-1-äthinyl -1-methylpropyl]-1,2-benzoldicarbonsäureester -(S)-α-methylbenzolmethanaminsalz (1:1), Smp. 162-165°.

## Beispiel 8

Die Mutterlauge aus Beispiel 7 wurde zu einem Oel eingedampft. Dieses wurde in 100 ml Diäthyläther aufgenommen und mit 100 ml 1,0 N HCl behandelt. Das Gemisch wurde 1/2 Stunde gerührt. Die Aetherschicht wurde abgetrennt und mit 2 x 100 ml 1,0 N HCl und 100 ml Wasser gewaschen. Die wässrige Phase wurde mit 100 ml Diäthyläther rückextrahiert. Die Aetherextrakte wurden vereinigt, über $MgSO_4$ getrocknet und filtriert. Diesem Filtrat wurden 50 ml 95% Aethanol, gefolgt von 5,2 g (0,043 Mol)

(R)-(+)-α-Methylbenzylamin zugesetzt. Die Lösung wurde 1 Stunde bei 0° gerührt. Durch Filtration erhielt man 18 g Kristalle. Diese wurden wie in Beispiel 7 beschrieben aus 50 ml Aethanol und 150 ml Diäthyläther umkristallisiert. Man erhielt 11,04 g (R)-[3-[2,5-Bis(acetyloxy)-3,4,6-trimethylphenyl]-1-äthinyl-1-methylpropyl]-1,2-benzoldicarbonsäureester -(R)-α-methylbenzolmethanaminsalz (1:1) (Ausbeute 32,8%), Smp. 163-166°, $[\alpha]_D^{25}$ = +9,6° (c = 1,03 $C_2H_5OH$).

## Beispiel 9

Ein Gemisch von 110 g des Salzes von Beispiel 7, 400 ml 1,0N HCl und 40 ml $CH_2Cl_3$-Aether (1:9) wurde 1 Stunde bei 25° gerührt. Es wurde dann mit 2 x 200 ml Aether extrahiert. Die vereinigten Extrakte wurden mit 1,0 N HCl und Wasser gewaschen und getrocknet. Nach Abdampfen des Aethers und Trocknen erhielt man 80,59 g (S)-(+)-[3-[2,5-Bis(acetyloxy)-3,4,6-trimethylphenyl] -1-äthinyl-1-methylpropyl]-1-2-benzoldicarbonsäureester, $[\alpha]_D^{25}$ = +15,95° (c = 0,96, Aethanol).

## Beispiel 10

(R)-(-)-[3-[2-Bis(acetyloxy)-3,4,6-trimethylphenyl]-1-äthinyl-1-methylpropyl]-1,2-benzoldicarbonsäureester

Dieses Produkt, $[\alpha]_D^{25}$ = -15,98° (Aethanol) wurde in Analogie zu Beispiel 9 ausgehend von (R)-[3-[2,5-Bis(acetyloxy)-3,4,6-trimethylphenyl]-1-äthinyl -1-methylpropyl]-1,2-benzoldicarbonsäureester -(R)-α-methylbenzolmethanaminsalz (1:1) erhalten.

## Beispiel 11

48 g (S)-[3-[2,5-Bis(acetyloxy)-3,4,6-trimethylphenyl]-1-äthinyl -1-methylpropyl]-1,2-benzoldicarbonsäureester wurden in 250 ml Methanol gelöst und mit Argon entgast. Dieser Lösung wurden 150 ml 6N NaOH zugetropft. Es wurde dann 1 Stunde unter Rückfluss erhitzt, auf 4° abgekühlt und mit 150 ml 5,6N $H_2SO_4$ auf pH 1,5 angesäuert. 200 ml Methanol und 0,2 g Eisen(III)chlorid-trihydrat wurden zugesetzt. Das Reaktionsgemisch wurde 18 Stunden unter Rückfluss erhitzt. Nach Abkühlen auf 25° wurden 500 ml Wasser zugesetzt. Das Gemisch wurde mit Diäthyläther extrahiert. Die Aetherextrakte wurden mit gesättigter Natriumbicarbonatlösung, 1N HCl und Wasser gewaschen und getrocknet. Danach wurde die Aetherlösung filtriert und das Filtergut mit Aether gewaschen. Der Aether wurde abgedampft. Kristallisation des entstandenen Feststoffs aus Toluol/Hexan ergab 15,62 g (S)-(+)-3,4-Dihydro-2-äthinyl-2,5,7,8-tetramethyl-2H-1-benzopyran-6-ol, Smp. 109-114°, $[\alpha]_D^{25}$ = +53.46° (c = 0,997, $CHCl_3$). Es wurden weitere 2,94 g dieses Produkts erhalten, Smp. 108-117°, $[\alpha]_D^{25}$ = +53,63° (c = 0,985, $CHCl_3$).

## Beispiel 12

(R)-(+)-3,4-Dihydro-2-äthinyl-2,5,7,8-tetramethyl-2H-1-benzopyran-6-ol.

Analog Beispiel 11 erhielt man 18,1 g dieses Produktes, Smp. 109-114°, $[\alpha]_D^{25}$ = +55,72° (c = 1,001, $CHCl_3$), in einer Ausbeute von 78% ausgehend von 48 g (R)-[3-[2,5-Bis(acetyloxy)-3,4,6-trimethylphenyl]-1-äthinyl -1-methylpropyl]-1,2-benzoldicarbonsäureester.

## Beispiel 13

(S)-3,4-Dihydro-2,5,7,8-tetramethyl-2-[(2-thienyl)äthinyl]-2H-1-benzopyran-6-ol.

Dieses Enantiomer wurde erhalten wie beschrieben in Beispiel 4 durch Umsetzung des Produkts von Beispiel 11 mit 2-Jodthiophen. Schmelzpunkt des Produktes nach Kristallisation aus Aether/Hexan 131-134°, $[\alpha]_D^{25}$ = +34,90 (c = 1,145 in MeOH).

## Beispiel 14

rac-3,4-Dihydro-2,5,7,8-tetramethyl-2-[(3-pyridinyl)äthinyl]-2H-1-benzopyran-6-ol.

Rac-3,4-Dihydro-2-äthinyl-2,5,7,8-tetramethyl-2H-1- benzopyran-6-ol, 4,6 g wurden analog Beispiel 4 mit 4,74 g 3-Brompyridin umgesetzt. Nach Chromatographie über Silicagel mit Aethylacetat in Methylenchlorid erhielt man das Produkt vom Smp. 172-175° nach Kristallisation aus Aethylacetat/Hexan.

## Beispiel 15

rac-3,4-Dihydro-2,5,7,8-tetramethyl-2-[(pyridinyl)äthinyl]-2H-1-benzopyran-6-ol.

Analog Beispiel 14 wurde dieses Produkt durch Reaktion von rac-3,4-Dihydro-2-äthinyl-2,5,7,8-tetramethyl-2H-1-benzopyran-6-ol mit 2-Brompyridin erhalten, Smp. 179-181° nach Kristallisation aus Aethylacetat.

## Beispiel 16

rac-[2-(Benzo[b]thiophen-3-yl)äthinyl]-3,4-dihydro-2,5,7,8-tetramethyl-2H-1-benzopyran-6-ol.

Ein Gemisch von 4,6 g rac-3,4-Dihydro-2-äthinyl-2,5,7,8-tetramethyl-2H -1-benzopyran-6-ol, 6,4 g 3-Brombenzo[b]thiophen, 0,4 g Triphenylphosphin, 95 mg Kupfer(I)jodid, 6 ml Triäthylamin und 150 ml Acetonitril wurde mit Argon entgast. 110 mg Palladiumacetat wurden zugesetzt. Es wurde 4 Tage bei Raumtemperatur gerührt. Das Lösungsmittel wurde abgedampft und der Rückstand zwischen Methylenchlorid und gesättigter Natriumbicarbonatlösung aufgeteilt. Die organische Phase wurde abgetrennt, getrocknet und eingedampft. Der Rückstand wurde über Silicagel mit Methylenchlorid/Hexan chromatographiert. Nach Kristallisation aus Aether/Hexan erhielt man 3,3 g Produkt, Smp. 123-124°.

## Beispiel 17

S(+)-[2-(Benzo[b]thiophen-3-yl)äthinyl]-3,4-dihydro-2,5,7,8-tetramethyl-2H-1-benzopyran-6-ol.

Dieser Enantiomer wurde wie weiter oben beschrieben ausgehend von dem Produkt von Beispiel 11 hergestellt. Das Produkt wurde wie in Beispiel 16 beschrieben isoliert, Smp. 58-61° (Methanol/Wasser), $[\alpha]_D^{25}$ = +31,58 (c = 1,045 in MeOH).

## Beispiel 18

R(-)-[2-(Benzo[b]thiophen-3-yl)äthinyl]-3,4-dihydro-2,5,7,8-tetramethyl-2H-1-benzopyran-6-ol.

Dieses Isomere des Produkts von Beispiel 17 wurde hergestellt durch Reaktion des Produkts von Beispiel 12 mit 3-Brombenzo[b]thiophen wie beschrieben in Beispiel 16. Smp. 58-61°, $[\alpha]_D^{25}$ = -30,11° (c = 1,002 in MeOH) nach Kristallisation aus Methanol/Wasser.

## Beispiel 19

rac-[2-(Benzo[b]thiopen-2-yl)äthinyl]-3,4-dihydro-2,5,7,8-tetramethyl-2H-1-benzopyran-6-ol.

Analog Beispiel 16 erhielt man aus rac-3,4-Dihydro-2-äthinyl-2,5,7,8-tetramethyl-2H -1-benzopyran-6-ol dieses Produkt von Smp. 120-123° nach Kristallisation aus Aether/Hexan.

## Beispiel 20

rac-2-[(3-Acetyloxy-4-methoxyphenyl)äthinyl]-3,4-dihydro-2,5,7,8-tetramethyl-2H-1-benzopyran-6-ol.

Analog Beispiel 4 wurden 1,15 g rac-3,4-Dihydro-2-äthinyl-2,5,7,8-tetramethyl-2H -1-benzopyran-6-ol mit 1.5 g (5-Jod-2-methoxyphenyl)acetat umgesetzt. Nach Chromatographie über Silicagel mit Aethylacetat in Methylenchlorid erhielt man 1,4 g Produkt, Smp. 142-144° nach Kristallisation aus Aethylacetat/Aether/Hexan. Das Ausgangsmaterial wurde wie in Beispiel 21 beschrieben hergestellt.

## Beispiel 21

(5-Jod-2-methoxyphenyl)acetat

Ein Gemisch von 12,5 g 2-Methoxyphenol, 20 ml Pyridin und 25 ml Essigsäureanhydrid wurde über Nacht bei Raumtemperatur stehen gelassen. Die Reaktanten wurden eingedampft, teilweise azeotropisch mit Toluol. Der Rückstand wurde in Eisessig gelöst. 10 ml Jodmonochlorid wurden zugesetzt. Nach 2 Stunden Rühren wurde mit Wasser verdünnt und mit Ueberschuss Natriumsulfit behandelt. Das Produkt wurde mit Methylenchlorid extrahiert und die Extrakte wurden mit Natriumsulfitlösung gewaschen, getrocknet und eingedampft. Der Rückstand wurde aus Aether/Hexan kristallisiert. Smp. des Produkts 89-90°.

## Beispiel 22

rac-3,4-Dihydro-2-[(3-methoxyphenyl)äthinyl]-2,5,7,8-tetramethyl-2H-1-benzopyran-6-ol.

Analog Beispiel 4 erhielt man aus 1,15 g rac-3,4-Dihydro-2-äthinyl-2,5,7,8-tetramethyl-2H -1-benzopyran-6-ol und 1,5 ml 3-Methoxyjodbenzol, 1,05 g Produkt, Smp. 108-112° nach Kristallisation aus Aether/Hexan.

## Beispiel 23

rac-5-[(3,4-Dihydro-6-hydroxy-2,5,7,8-tetramethyl-2H-1-benzopyran-2-yl)äthinyl]-2-hydroxybenzosäuremethyl ester.

Analog Beispiel 4 wurde dieses Produkt hergestellt aus rac-3,4-Dihydro-2-äthinyxl-2,5,7,8-tetramethyl-2H -1-benzopyranol und 2-Hydroxy-5-jodbenzoesäuremethylester [Chim. Ther. 2, (1967), 73], Schmelzpunkt des Produkts nach Kristallisation aus Aethylacetat/Hexan 151-153°.

## Beispiel 24

rac-3,4-Dihydro-2,5,7,8-tetramethyl-2-{[4-(3-pyridinyl)phenyl]äthinyl}-2H-1-benzopyran-6-ol.

Ein Gemisch von 1,15 g rac-3,4-Dihydro-2-äthinyl-2,5,7,8-tetramethyl-2H -1-benzopyran-6-ol, 1,14 g 3-(4-Jodphenyl)pyridin, 0,2 g Triphenylphosphin, 50 mg Kupfer(I)jodid, 1,5 ml Triäthylamin und 20 ml Acetonitril wurde mit Argon entgast. 75 mg Palladiumacetat wurden zugesetzt. Das Reaktionsgemisch wurde zwischen Methylenchlorid und Wasser verteilt. Die organische Phase wurde getrocknet und eingedampft. Der Rückstand wurde aus Aethylacetat kristallisiert und aus Methanol/Aethylacetat umkristallisiert. Smp. des Produkts 190-192°.

Das Ausgangsmaterial wurde wie in Beispiel 25 beschrieben hergestellt.

## Beispiel 25

3-(4-Jodphenyl)pyridin.

Ein Gemisch von 1,7 g 3-(4-Aminophenyl)pyridin [J. Agric. Food Chem. 30 (1982) 957], 17 ml Eisessig und 1,5 ml Trifluoressigsäure wurde in Eiswasser abgekühlt. 0,8 g Natriumnitrit wurden zugesetzt. Dann wurden 5 g Natriumjodid und 3 g Natriumacetat zugesetzt. Nach Zusatz von Wasser wurde das Reaktionsgemisch zwischen Methylenchlorid und einer Natriumcarbonatlösung verteilt. Nach Chromatographie der organischen Phase über Silicagel mit Aethylacetat in Methylenchlorid wurde das Produkt erhalten. Smp. 112-115°.

## Beispiel 26

rac-3,4-Dihydro-2,5,7,8-tetramethyl-2-{[5-(2-pyridinyl)-2-thienyl]äthinyl}-2H-1-benzopyran-6-ol.

Analog Beispiel 24 wurde dieses Produkt aus 2,3 g rac-3,4-Dihydro-2-äthinyl-2,5,7,8-tetramethyl-2H -1-benzopyran-6-ol und 3,6 g 2-(5-Brom-2-thienyl)pyridin hergestellt. Nach Eindampfen unter vermindertem Druck wurde der Rückstand zwischen Methylenchlorid und Wasser verteilt. Die organische Schicht wurde getrocknet und eingedampft und der Rückstand wurde über Silicagel mit Aethylacetat in Methylenchlorid chromatographiert. Die gereinigten Fraktionen wurden eingedampft und der Rückstand wurde aus Aether/Hexan kristallisiert. Man erhielt 2 g Kristalle, Smp. 159-162°. Das Ausgangsarylhalogenid wurde wie in Beispiel 27 beschrieben hergestellt.

## Beispiel 27

2-(5-Brom-2-thienyl)pyridin.

16 g Brom wurden einer Lösung von 8,05 g 2-(2-Thienyl)pyridin in 250 ml Methylenchlorid zugesetzt. Nach Rühren wurde das Reaktionsgemisch mit einer Natriumcarbonatlösung gewaschen, getrocknet und eingedampft. Nach Kristallisation aus Aethanol erhielt man 9,5 g Produkt, Smp. 85-87°.

## Beispiel 28

rac-3-[(3,4-Dihydro-6-hydroxy-2,5,7,8-tetramethyl-2H-1-benzopyran-2-yl)äthinyl]-4-hydroxy-5-methoxybenzaldehyd.

Analog Beispiel 1 erhielt man durch Reaktion von 2,3 g rac-3,4-Dihydro-2-äthinyl-2,5,7,8-tetramethyl-2H-1-benzopyran-6-ol mit 4,17 g 5-Jodvanillin das obige Produkt, Smp. 158-160° nach Kristallisation aus Aether Hexan und Umkristallisation aus Aethylacetat/Hexan.

## Beispiel 29

rac-3-[(3,4-Dihydro-6-hydroxy-2,5,7,8-tetramethyl-2H-1-benzopyran-2-yl)äthinyl]-4-hydroxy-5-methoxybenzylalkohol.

Analog Beispiel 1 erhielt man durch Reaktion von rac-3,4-Dihydro-2-äthinyl-2,5,7,8-tetramethyl-2H -1-benzopyran-6-ol mit 3-Jod-4-hydroxy-5-methoxybenzylalkohol (Monatsh. Chem. 103, 1972, 1178) das obige Produkt, Smp. 160-163° nach Kristallisation aus Aether/Hexan.

## Beispiel 30

rac-3-[(3,4-Dihydro-6-hydroxy-2,5,7,8-tetramethyl-2H-1-benzopyran-2-yl)äthinyl]-4-hydroxy-5-methoxybenzaldehydoxim.

Analog Beispiel 1 erhielt man durch Reaktion von rac-3,4-Dihydro-2-äthinyl-2,5,7,8-tetramethyl-2H -1-benzopyran-6-ol mit 3-Jod-4-hydroxy-5-methoxybenzaldehydoxim das obige Produkt, Smp. 210-213° nach Kristallisation aus Methanol/Aethylacetat.

## Beispiel 31

rac-2-[(5-Butyl-2-thienyl)äthinyl]-3,4-dihydro-2,5,7,8-tetramethyl-2H-1-benzopyran-6-ol.

Analog Beispiel 14 erhielt man durch Reaktion von 2,3 g rac-3,4-Dihydro-2-äthinyl-2,5,7,8-tetramethyl-2H -1-benzopyran-6-ol mit 2,4 g 2-Brom-5-butylthiophen das obige Produkt, Smp. 80-82° nach Kristallisation aus Petroläther. Das Ausgangsmaterial wird wie in Beispiel 32 beschrieben hergestellt.

## Beispiel 32

2-Brom-5-butylthiophen

15,63 g Brom wurden einer Lösung von 15,2 g 2-Butylthiophen in 400 ml Chloroform zugesetzt. Nach 10 Minuten Rühren wurde das Gemisch mit einer Natriumcarbonatlösung gewaschen. Die organische Schicht wurde getrennt, getrocknet und eingedampft. Das entstandene Oel wurde unter Hochvakuum destilliert.

## Beispiel 33

rac-3,4-Dihydro-2,5,7,8-tetramethyl-2-(3-chinolinyläthinyl)-2H-1-benzopyran-6-ol.

Analog Beispiel 14 wurde durch Reaktion von 2,3 g rac-3,4-Dihydro-2-äthinyl-2,5,7,8-tetramethyl-2H-1-benzopyran-6-ol, mit 3,12 g 3-Bromchinolin das obige Produkt, Smp. 199-203° nach Kristallisation aus Aethylacetat, erhalten.

## Beispiel 34

rac-3,4-Dihydro-2-[(4-isochinolinyl)äthinyl]-2,5,7,8-tetramethyl-2H-1-benzopyran-6-ol.

Analog Beispiel 33, jedoch unter Verwendung von 4-Bromisochinolin, erhielt man das obige Produkt, Smp. 227-230° nach Kristallisation aus Toluol.

## Beispiel 35

rac-2-{(3-(Trifluormethyl)phenyl]äthinyl}-3,4-dihydro-2,5,7,8-tetramethyl-2H-1-benzopyran-6-ol.

Analog Beispiel 26 erhielt man durch Reaktion von rac-3,4-Dihydro-2-äthinyl-2,5,7,8-tetramethyl-2H-1-benzopyran-6-ol mit 3-Trifluormethylbrombenzol das obige Produkt, Smp. 81-83°.

## Beispiel 36

rac-2-[(3,4-Difluorphenyl)äthinyl]-3,4-dihydro-2,5,7,8-tetramethyl-2H-1-benzopyran-6-ol.

Analog Beispiel 26 erhielt man durch Reaktion von rac-3,4-Dihydro-2-äthinyl-2,5,7,8-tetramethyl-2H -1-benzopyran-6-ol mit 1-Brom-3,4-difluorbenzol das obige Produkt, Smp. 109-112° nach Kristallisation aus Petroläther.

## Beispiel 37

rac-3,4-Dihydro-2-[(4-hydroxy-3-propylphenyl)äthinyl]-2,5,7,8-tetramethyl-2H-1-benzopyran-6-ol.

Analog Beispiel 1 erhielt man durch Reaktion von rac-3,4-Dihydro-2-äthinyl-2,5,7,8-tetramethyl-2H -1-benzopyran-6-ol mit 4-Jod-2-propylphenol das obige Produkt, Smp. 154-156°. Das Ausgangsmaterial wurde wie in Beispiel 38 beschrieben hergestellt.

## Beispiel 38

4-Jod-2-propylphenol.

Ein Gemisch von 6,8 g 2-Propylphenol, 8,25 g Natriumjodid, 175 ml Acetonitril und 40 ml Wasser wurde über Eis abgekühlt. 6 g t-Butylhypochlorit wurden zugesetzt. Nach Rühren bei 0° wurden 500 ml Aethylacetat zugesetzt und das Gemisch wurde mit wässrigem Natriumsulfat gewaschen. Die organische Schicht wurde getrocknet und eingedampft und der Rückstand wurde über Silicagel mit Methylenchlorid/Hexan chromatographiert. Man erhielt das Produkt nach Kristallisation aus Petroläther, Smp. 54-56°.

## Beispiel 39

rac-3,4-Dihydro-2,5,7,8-tetramethyl-2-[(1-naphthyl)äthinyl]-2H-1-benzopyran-6-ol.

Analog Beispiel 1 erhielt man durch Reaktion von rac-3,4-Dihydro-2-äthinyl-2,5,7,8-tetramethyl-2H -1-benzopyran-6-ol mit 1-Jodnaphthalen das obige Produkt, Smp. 127-128° nach Kristallisation aus Hexan und Umkristallisation aus Petroläther.

## Beispiel 40

rac-2-[(2-Aminophenyl)äthinyl]-3,4-dihydro-2,5,7,8-tetramethyl-2H-1-benzopyran-6-ol.

Analoge Beispiel 26 erhielt man durch Reaktion des rac-3,4-Dihydro-2-äthinyl-2,5,7,8-tetramethyl-2H -1-benzopyran-6-ols mit 2-Jodanilin das obige Produkt, Smp. 110-112° nach Kristallisation aus Cyclohexan.

## Beispiel 41

rac-2-{[3-Acetyloxy-4-(phenylmethoxy)phenyl]äthinyl}-3,4-dihydro-2,5,7,8-tetramethyl-2H-1-benzopyran-6-ol.

Analog Beispiel 26 erhielt man durch Reaktion von rac-3,4-Dihydro-2-äthinyl-2,5,7,8-tetramethyl-2H-1-benzopyran-6-ol mit 5-Jod-2-(phenylmethoxy)phenylacetat das obige Produkt, Smp. 117-120° nach Kristallisation aus Aether/Hexan. Das Ausgangsmaterial wurde wie folgt hergestellt.

## Beispiel 42

5-Jod-2-(Phenylmethoxy)phenolacetat.

Ein Gemisch von 8 g 2-(phenylmethoxy)phenol, 20 ml Pyridin und 30 ml Essigsäureanhydrid wurde über Nacht bei Raumtemperatur stehen gelassen. Die Reagenzien wurden unter vermindertem Druck abgedampft, teilweise azeotropisch mit Xylol und dann mit Tetrachlorkohlenstoff. Der Rückstand wurde in Essigsäure aufgenommen und die Lösung eisgekühlt. 19 g Jodmonochlorid wurden zugesetzt und das Gemisch wurde auf Eiswasser geschüttet, mit Natriumsulfit behandelt und mit Methylenchlorid extrahiert. Die Extrakte wurden mit 10%iger Natriumcarbonatlösung gewaschen, getrocknet und eingedampft. Der

Rückstand wurde aus Hexan kristallisiert und aus Methanol umkristallisiert. Schmelzpunkt des Produkts 71-73°.

## Beispiel 43

rac-3,4-Dihydro-2-{[3-hydroxy-4-(phenylmethoxy)phenyl]äthinyl}-2,5,7,8-tetramethyl-2H-1-benzopyran-6-ol.

0,12 g Natriumhydroxid wurden einer Lösung von 0,47 g rac-2-{[3-Acetyloxy-4-(phenylmethoxy)phenyl]-äthinyl}-3,4-dihydro-2,5,7,8-tetramethyl-2H-1-benzopyran-6-ol in 10 ml Methanol zugesetzt. Das Gemisch wurde unter Argon gerührt und dann mit Eisessig angesäuert und zwischen Methylenchlorid und gesättigten Natriumbicarbonatlösung verteilt. Die organische Schicht wurde abgetrennt, getrocknet und über Silicagel filtriert. Das Filtrat wurde eingedampft und der Rückstand aus Aether/Hexan kristallisiert. Schmelzpunkt des Produktes 103-106°.

## Beispiel 44

rac-2,2,2-Trifluor-N-{2-[(3,4-dihydro-6-hydroxy-2,5,7,8-tetramethyl-2H-1-benzopyran-2-yl)äthinyl]-phenyl}acetamid.

0,4 ml Trifluoressigsäureanhydrid wurden einer Lösung von 0,3 g rac-2-[(2-Aminophenyl)äthinyl]-3,4-dihydro-2,5,7,8-tetramethyl-2H -1-benzopyran-6-ol in 25 ml Methylenchlorid zugesetzt. Dem Gemisch wurde wässriges Natriumcarbonat zugesetzt. Die organische Schicht wurde abgetrennt, getrocknet und eingedampft. Der Rückstand wurde in Hexan/Aether gelöst, filtriert und kristallisiert. Schmelzpunkt des Produkts 116-118°.

## Beispiel 45

rac-3,4-Dihydro-6-[(3,4-dihydro-6-hydroxy-2,5,7,8-tetramethyl-2H-1-benzopyran-2-yl)äthinyl]-4-(trifluoracetyl)-2H-1,4-benzoxazin.

Wie beschrieben in Beispiel 14 erhielt man durch Reaktion von 1,15 g rac-3,4-Dihydro-2-äthinyl-2,5,7,8-tetramethyl-2H-1-benzopyran-6-ol mit 4-(Trifluoracetyl)-3,4-dihydro-6-jod-2H-1,4-benzoxazin 1,95 g obigen Produkts, Smp. 153-155° nach Kristallisation aus Methanol. Das Ausgangsmaterial wurde wie folgt hergestellt.

## Beispiel 46

4-(Trifluoracetyl)-3,4-dihydro-6-jod-2H-1,4-benzoxazin.

5,6 ml Trifluoressigsäureanhydrid wurden einer Lösung von 4 g 3,4-Dihydro-2H-1,4-benzoxazin (Nagoya Shiritsu Daigaku Takugakubu Kenkyu Nempo 17, 1969, 50) in 50 ml Methylenchlorid zugesetzt. Nach Zugabe von wässrigem Natriumcarbonat wurde das Gemisch eisgekühlt. Die organische Schicht wurde abgetrennt, mit Salzwasser gewaschen, getrocknet und eingedampft. Kristallisation aus Hexan ergab 3,4-Dihydro-4-(trifluoracetyl)-2H-1,4-benzoxazin, Smp. 58-60°.

Ein Gemisch von 0,5 g dieses Benzoxazins, 20 ml Eisessig und 5 ml Methylenchlorid wurde auf 10° abgekühlt. Eine Lösung von 1 ml Jodmonochlorid in 1 ml Essigsäure wurde zugesetzt. Das Gemisch wurde

mit Wasser und wässrigem Natriumbisulfit verdünnt. Das Produkt wurde mit Aether extrahiert. Die Extrakte wurden gewaschen, getrocknet und eingedampft. Schmelzpunkt des Produkts 78-80° nach Kristallisation aus Hexan.

Beispiel 47

rac-2-[(3,4-Dihydro-2H-1,4-benzoxazin-2-yl)äthinyl]-3,4-dihydro-2,5,7,8-tetramethyl-2H-1-benzopyran-6-ol-hydrochlorid.

Eine Lösung von 0,3 g rac-3,4-Dihydro-6-[(3,4-dihydro-6-hydroxy-2,5,7,8 -tetramethyl-2H-1-benzopyran-2-yl)äthinyl] -4-(trifluoracetyl)-2H-1,4-benzoxazin in 25 ml Methanol wurde mit Argon entgast und mit 3N Natriumhydroxidlösung behandelt. Das Gemisch wurde mit Essigsäure angesäuert und eingedampft. Der Rückstand wurde zwischen Methylenchlorid und gesättigtem wässrigem Natriumcarbonat verteilt. Die organische Schicht wurde getrocknet und einge dampft. Der Rückstand wurde mit äthanolischer Salzsäure behandelt und durch Zusatz von Aether kristallisiert. Schmelzpunkt des 0,33 Moläquivalente Wasser enthaltenden Produkts 150-165°, Zersetzung.

Beispiel 48

6-[(3,4-Dihydro-6-hydroxy-2,5,7,8-tetramethyl-2H-1-benzopyran-2-yl)äthinyl]-4-methyl-2H-1,4-benzoxazin-(4H)-on.

Analog Beispiel 26 erhielt man durch Reaktion von 1,15 g rac-3,4-Dihydro-2-äthinyl-2,5,7,8-tetramethyl-2H -1-benzopyran-6-ol mit 1,6 g 6-Jod-4-methyl-2H-1,4-benzoxazin-3(4H)-on das obige Produkt, Smp. 185-188° nach Kristallisation aus Methanol. Das Ausgangsmaterial wurde wie folgt hergestellt.

Beispiel 49

6-Jod-4-methyl-2H-1,4-benzoxazin-3(4H)-on.

Eine Lösung von 5 g 4-Methyl-2H-1,4-benzoxazin-3(4H)-on in 100 ml Essigsäure und 25 ml Methylenchlorid wurde auf 10° abgekühlt. Nach Zusatz einer Lösung von 15 ml Jodmonochlorid in 15 ml Essigsäure wurde das Reaktionsgemisch mit Wasser verdünnt und überschüssiges Reagens mit Natriumsulfit reduziert. Das Produkt wurde mit Aether extrahiert, die Extrakte wurden mit wässrigem Natriumcarbonat gewaschen, getrocknet und eingedampft. Schmelzpunkt des Produkts 146-148° nach Kristallisation aus Aether.

Beispiel 50

rac-3,4-Dihydro-2-[(2-hydroxyphenyl)äthinyl]-2,5,7,8-tetramethyl-2H-1-benzopyran-6-ol und rac-2-(2-Benzofuranyl)-3,4-dihydro-2,5,7,8-tetramethyl-2H-1-benzopyran-6-ol.

Analog Beispiel 14 erhielt man durch Reaktion des rac-3,4-Dihydro-2-äthinyl-2,5,7,8-tetramethyl-2H-1-benzopyran-6-ol mit 2-Jodphenol ein Gemisch der obigen Verbindungen. Nach Chromatographie über Silicagel mit Methylenchlorid erhielt man zunächst das weniger polare 2-(2-Benzofuranyl)-3,4-dihydro-2,5,7,8-tetramethyl-2H -1-benzopyran-6-ol, Smp. 90-93° nach Kristallisation aus Aether/Hexan und das rac-3,4-Dihydro-2-[(2-hydroxyphenyl)äthinyl]-2,5,7,8-tetramethyl-2H-1-benzopyran-6-ol, Smp. 123-125° nach

Kristallisation aus Aether/Hexan und Umkristallisation aus Hexan.

Beispiel 51

rac-3,4-Dihydro-2-(5-methylfuro[3,2-b]pyridin-yl)-2,5,7,8-tetramethyl-2H-1-benzopyran-6-ol.

Analog Beispiel 26 erhielt man durch Reaktion von rac-3,4-Dihydro-2-äthinyl-2,5,7,8-tetramethyl-2H -1-benzopyran-6-ol mit 2-Jod-3-hydroxy-6-methylpyridin das obige Produkt, Smp. 115-118° nach Kristallisation aus Aether Hexan.

Beispiel 52

rac-3,4-Dihydro-2-(furo[3,2-b]pyridin-2-yl)-2,5,7,8-tetramethyl-2H-1-benzopyran-6-ol.

Analog Beispiel 26 wurde durch Reaktion von rac-3,4-Dihydro-2-äthinyl-2,5,7,8-tetramethyl-2H-1-benzopyran-6-ol mit 2-Brom-3-hydroxypyridin das obige Produkt erhalten, Smp. 168-169° nach Kristallisation aus Aethylacetat/Hexan.

Beispiel 53

rac-1-[2-(3,4-Dihydro-6-hydroxy-2,5,7,8-tetramethyl-2H-1-benzopyran-2-yl)-6-hydroxy-7-propylbenzofuran-5-yl]äthanon.

Analog Beispiel 26 erhielt man durch Reaktion von rac-3,4-Dihydro-2-äthinyl-2,5,7,8-tetramethyl-2H -1-benzopyran-6-ol mit 1-(2,4-Dihydroxy-5-jod-3-propylphenyl)äthanon [USP 4,252,818] das obige Produkt, Smp. 121-124° nach Kristallisation aus Aether/Hexan.

Beispiel 54

rac-2-(3,4-Dihydro-6-hydroxy-2,5,7,8-tetramethyl-2H-1-benzopyran-2-yl)-7-methoxy-5-benzofurancarboxaldehyd.

Analog Beispiel 26 erhielt man durch Reaktion von rac-3,4-Dihydro-2-äthinyl-2,5,7,8-tetramethyl-2H -1-benzopyran-6-ol mit 3-Jod-4-hydroxy-5-methoxybenzaldehyd das obige Produkt, Smp. 124-127° nach Kristallisation aus Aethylacetat/Hexan.

Beispiel 55

rac-2-(3,4-Dihydro-6-hydroxy-2,5,7,8-tetramethyl-2H-1-benzopyran-2-yl)-7-methoxybenzofuran-5-methanol.

Ein Gemisch von 0,2 g des Produkts von Beispiel 54 und 20 mg Natriumborhydrid in 5 ml Aethanol wurde unter Stickstoff gerührt. Es wurde zwischen Methylenchlorid und gesättigter Natriumbicarbonatlösung verteilt. Die organische Schicht wurde abgetrennt, getrocknet und eingedampft. Nach Kristallisation aus

Aether/Hexan erhielt man das obige Produkt, Smp. 122-125°.

Beispiel 56

3,4-Dihydro-2,5,7,8-tetramethyl-2-(2-phenyläthyl)-2H-1-benzopyran-6-ol.

Ein Gemisch von 1.3 g des Produkts von Beispiel 1, 0,3 g 5% Pd/C, 50 ml THF und 50 ml Aethanol wurde unter Normaldruck hydriert. Der Katalysator wurde abfiltriert und das Filtrat eingedampft. Kristallisation des Rückstands aus Hexan ergab 0,9 g des Produkts, Smp. 96-98°.

Beispiel 57

rac-3,4-Dihydro-2,5,7,8-tetramethyl-2-[2-(2-thienyl)äthyl]-2H-1-benzo-pyran-6-ol.

Analog Beispiel 26 erhielt man durch Hydrierung von rac-3,4-Dihydro-2,5,7,8-tetramethyl-2-[2-(2-thienyl}äthinyl]-2H-2-benzopyran-6-ol das obige Produkt, Smp. 95-98° nach Kristallisation aus Hexan.

Beispiel 58

rac-3,4-Dihydro-2,5,7,8-tetramethyl-2-[2-(2-pyridinyl)äthyl]-2H-1-benzopyran-6-ol.

Analog Beispiel 56 erhielt man dieses Produkt durch Hydrierung von rac-3,4-Dihydro-2,5,7,8-tetramethyl-2-[2-(2-pyridinyl)äthinyl]- 2H-1-benzopyran-6-ol. Schmnelzpunkt des Produkts 141-142° nach Kristallisation aus Aethylacetat/Hexan.

Beispiel 59

rac-3,4-Dihydro-2,5,7,8-tetramethyl-2-[2-(3-pyridinyl)äthyl]-2H-1-benzopyran-6-ol.

Analog Beispiel 56 erhielt man das obige Produkt, Smp. 127-129° nach Kristallisation aus Aethylacetat/Hexan.

Beispiel 60

rac-2-{2-[3-(Acetyloxy)-4-methoxyphenyl]äthyl}-3,4-dihydro-2,5,7,8-tetramethyl-2H-1-benzopyran-6-ol.

Dieses Produkt, Smp. 110-113° (Aether/Hexan) wurde durch Hydrierung von 0,7 g rac-2-{2-[3-Acetyloxy)-4-methoxyphenyl]äthinyl} -3,4-dihydro-2,5,7,8-tetramethyl-2H-1-benzopyran-6-ol in Gegenwart von 0,2 g 5% Pd/C in 50 ml Aethanol erhalten.

Beispiel 61

rac-4-[2-(3,4-Dihydro-6-hydroxy-2,5,7,8-tetramethyl-2H-1-benzopyran-2-yl)äthyl]-1,2-dihydroxybenzol.

Dieses Produkt, Smp. 167-170° wurde erhalten durch Hydrierung von 1 g rac-3,4-Dihydro-2-{[3-hydroxy-4-(phenylmethoxy)phenyl]äthinyl}-2,5,7,8-tetramethyl-2H-1-benzopyran-6-ol in Gegenwart von 0,5 g 5% Pd/C in 100 ml Aethanol und 10 ml Eisessig.

Beispiel 62

rac-3,4-Dihydro-2-[2-(3-methoxyphenyl)äthyl]-2,5,7,8-tetramethyl-2H-1-Benzopyran-6-ol.

Dieses Produkt, Smp. 105-107° (Aether Hexan) wurde erhalten analog Beispiel 56 durch Hydrierung von rac-3,4- Dihydro-2-[2-(3-methoxyphenyl)äthinyl]-2,5,7,8-tetramethyl-2H-1-benzopyran-6-ol.

Beispiel 63

rac-5-[2-(3,4-Dihydro-6-hydroxy-2,5,7,8-tetramethyl-2H-1-benzopyran-2-yl)äthyl]-2-hydroxybenzoesäuremethyl ester.

Durch Hydrierung von 0,7 g rac-5-[2-(3,4-Dihydro-6-hydroxy-2,5,7,8-tetramethyl-2H -1-benzopyran-2-yl)-äthinyl]-2-hydroxybenzoesäuremethylester erhielt man 0,5 g dieses Produkts, Smp. 108-110° (Aether Hexan).

Beispiel 64

rac-3,4-Dihydro-2,5,7,8-tetramethyl-2-{2-[4-(3-pyridinyl)phenyl]äthyl}-2H-1-benzopyran-6-ol.

Analog Beispiel 56 erhielt man dieses Produkt, Smp. 175-178° (Aethylacetat) durch Hydrierung von rac-3,4-Dihydro-2,5,7,8-tetramethyl -2-{2-[4-(3-pyrindinyl)phenyl]äthinyl}-2H -1-benzopyran-6-ol.

Beispiel 65

rac-3,4-Dihydro-2,5,7,8-tetramethyl-2-{2-[5-(2-pyridinyl)-2-thienyl]äthyl}-2H-1-benzopyran-6-ol.

Analog Beispiel 56 erhielt man dieses Produkt, Smp. 140-142° (Aethanol) durch Hydrierung von rac-3,4-Dihydro-2,5,7,8-tetramethyl-2-{2-[5-(2-pyridinyl) -2-thienyl]äthinyl}-2H-1-benzopyran-6-ol.

Beispiel 66

rac-2-[2-(5-Butyl-2-thienyl)äthyl]-3,4-dihydro-2,5,7,8-tetramethyl-2H-1-benzopyran-6-ol.

Analog Beispiel 56 erhielt man dieses Produkt, Smp. 83-85° (Petroläther) durch Hydrierung von rac-2-[2-(5-Butyl-2-thienyl)äthinyl]-3,4-dihydro-2,5,7,8 -tetramethyl-2H-1-benzopyran-6-ol.

## Beispiel 67

rac-4-[2-(3,4,-Dihydro-6-hydroxy-2,5,7,8-tetramethyl-2H-1-benzopyran-2-yl)äthyl]-1,2-benzoldiol-2-acetat.

Ein Gemisch von 0,94g rac-2{[3-Acetyloxy-4-(phenylmethoxy)phenyl]äthinyl}-3,4 -dihydro-2,5,7,8-tetramethyl-2H-1-benzopyran-6-ol, 0,5 g 5% Pd/C, 50 ml Aethanol und 2 ml Eisessig wurde 4 Stunden unter Normaldruck hydriert. Der Katalysator wurde abfiltriert und das Filtrat eingedampft. Der Rückstand wurde zwischen Methylenchlorid und gesättigtem Natriumbicarbonat verteilt. Die organische Schicht wurde getrocknet und eingedampft. Der Rückstand wurde aus Aether/Hexan kristallisiert. Smp. des Produkts 139-141°.

## Beispiel 68

rac-{2-[5-(Aminomethyl)-2-hydroxy-3-methoxyphenyl]äthyl}-3,4-dihydro-2,5,7,8-tetramethyl-2H-1-benzopyran-6-ol-hydrochlorid.

Ein Gemisch von 0,3 g rac-3[(3,4-Dihydro-6-hydroxy-2,5,7,8-tetramethyl-2H -1-benzopyran-2-yl)äthinyl]-4-hydroxy -5-methoxybenzaldehydoxim, 0,15 g Pd/C und 20 ml Aethanol wurde unter Normaldruck hydriert. Der Katalysator wurde abfiltriert und der Rückstand mit äthanolischer Salz säure behandelt und durch Zusatz von Aethylacetat und Aether kristallisiert. Nach Umkristallisation aus Methanol/Aethylacetat erhielt man das Produkt, Smp. 164-167° (Zersetzung).

## Beispiel 69

rac-6-Acetyloxy-3,4-dihydro-2,5,7,8-tetramethyl-2-(phenyläthinyl)-2H-1-benzopyran.

Ein Gemisch von 0,3 g rac-3,4-Dihydro-2,5,7,8-tetramethyl-2-(phenyläthinyl)-2H-1-benzopyran-6-ol, 7 ml Pyridin und 0,5 ml Essigsäureanhydrid wurde über Nacht stehen gelassen. Es wurde unter vermindertem Druck abgedampft, teilweise azeotropisch mit Toluol. Kristallisation aus Hexan ergab 0,21 g Produkt, Smp. 123-125°.

## Beispiel 70

rac-6-Acetyloxy-2-[(benzo[b]thiophen-3-yl)äthinyl]-3,4-dihydro-2,5,7,8-tetramethyl-2H-1-benzopyran.

Dieses Produkt, Smp. 99-101° (Hexan) wurde analog Beispiel 69 erhalten ausgehend von rac-2-[-(Benzo[b]thiophen-3-yl)äthinyl]-3,4-dihydro-2,5,7,8-tetramethyl-2H-1-benzopyran-6-ol.

## Beispiel 71

rac-(Z)-3,4-Dihydro-2,5,7,8-tetramethyl-2-[(2-pyridinyl)äthinyl]-2H-1-benzopyran-6-ol-hydrochlorid.

Ein Gemisch von 0,3 g rac-3,4-Dihydro-2,5,7,8-tetramethyl-2-[(2-pyridinyl)äthinyl]- 2H-1-benzopyran-6-ol, 0,15 g 5% Pd/C, 20 ml THF, 10 ml Aethanol und 0,2 ml Thiophen wurde unter Normaldruck hydriert. Der

Katalysator wurde abfiltriert und der Rückstand über Silicagel mit Hexan/THF chromatographiert. Die das Produkt enthaltenden Fraktionen wurden eingedampft. Der Rückstand wurde in das Hydrochlorid übergeführt, Smp. 218-220° nach Kristallisation aus Methanol/Aether.

Beispiel 72

rac-6-Acetyloxy-2-[(benzo[b]thiophen-3-yl)äthinyl]-3,4-dihydro-2,5,7,8-tetramethyl -2H-1-benzopyran.

Ein Gemisch von 0,544 g rac-6-Acetyloxy-2-äthinyl-3,4-dihydro-2,5,7,8-tetramethyl-2H-1-benzopyran, 0,64 g 3-Brombenzothiophen, 90 mg Triphenylphosphin, 20 mg Kupfer(I)jodid, 2 ml Triäthylamin und 40 ml DMF wurde mit Argon entgast. Nach Zusatz von 30 mg Palladiumacetat wurde das Gemisch auf 95° erhitzt. Das Reaktionsgemisch wurde zwischen gesättigtem Natriumbicarbonat und Toluol aufgeteilt. Die organische Phase wurde gewaschen, getrocknet und eingedampft. Der Rückstand wurde über Silicagel mit Toluol chromatographiert. Nach Kristallisation aus Hexan erhielt man das Produkt, Smp. 96-99°.

Beispiel 73

In an sich bekannter Weise wurden Tabletten folgender Zusammensetzung durch Feuchtgranulierung hergestellt:

| | Bestandteil | mg/Tablette | |
|---|---|---|---|
| 1. | rac-[2-(Benzo[b]thiophen-3-yl)äthinyl]-3,4-dihydro-2,5,7,8-tetramethyl-2H-1-benzopyran-6-ol | 100 | 500 |
| 2. | Lactose | 30 | 150 |
| 3. | Vorgelatinisierte Stärke | 6 | 30 |
| 4. | Mikrokristalline Cellulose | 30 | 150 |
| 5. | Magnesiumstearat | 1 | 6 |
| | Total | 167 | 836 |

Beispiel 74

In an sich bekannter Weise wurden Kapseln folgender Zusammensetzung hergestellt:

| | Bestandteil | mg/Kapsel | |
|---|---|---|---|
| 1. | rac-[2-(Benzo[b]thiophen-3-yl)äthinyl]-3,4-dihydro-2,5,7,8-tetramethyl-2H-1-benzopyran-6-ol | 100 | 500 |
| 2. | Vorgelatinisierte Maisstärke | 8 | 40 |
| 3. | Modifizierte Stärke | 4 | 20 |
| 4. | Talk | 4 | 20 |
| 5. | Magnesiumstearat | 1 | 2 |
| | Total | 117 | 582 |

Beispiel 75

In an sich bekannter Weise wurde ein Inhalationsaerosol in form einer Suspension folgender Zusammensetzung hergestellt:

| | Bestandteil | Gewichtsprozent |
|---|---|---|
| 1. | rac-[2-(Benzo[b]thiophen-3-yl)äthinyl]-3,4-dihydro-2,5,7,8-tetramethyl-2H-1-benzopyran-6-ol (mikronisiert) | 1.0 |
| 2. | Sorbitantrioleat | 0.5 |
| 3. | Chlorfluorkohlenstoffe | 98.5 |

Beispiel 76

In an sich bekannter Weise wurde eine Crème folgender Zusammensetzung hergestellt:

| Bestandteil | | g/kg | mögliche Variationen |
|---|---|---|---|
| rac-[2-(Benzo[b]thiophen-3-yl)äthinyl]-3,4-dihydro-2,5,7,8-tetramethyl-2H-1-benzopyran-6-ol | | 5.150 | ----- |
| Glycerylmonostearat | | 100.00 | 80-120 |
| Polysorbat 60 | | 20.00 | 15-25 |
| Cetylalkohol | | 50.00 | 40-60 |
| Petrolatum | | 70.00 | 50-90 |
| Methylparaben | | 1.50 | 1.25-1.75 |
| Propylparaben | | 0.50 | 0.4-0.6 |
| Propyleneglykol | | 200.00 | 150-250 |
| Wasser | | 568.05 | 475-575 |
| | Total | 1,015.20 | |

**Ansprüche**

1. Benzopyranolderivate der Formel

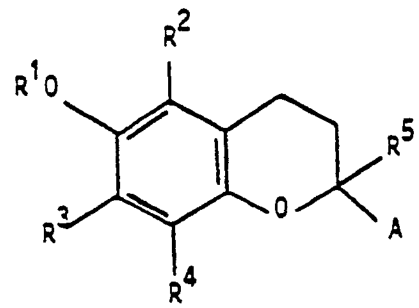

I

worin A -C≡C-$R^6$, -$CH_2CH_2$-$R^7$ oder

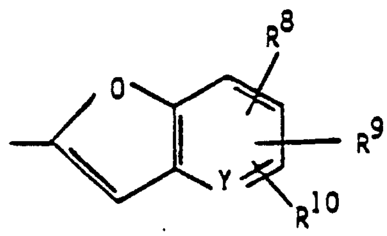

A'

und $R^1$ Wasserstoff oder nieder-Alkanoyl,
$R^2$, $R^3$ und $R^4$ Wasserstoff oder nieder-Alkyl,
$R^5$ nieder-Alkyl,
$R^6$ und $R^7$ ein heteroaromatisches Radikal oder ein aromatisches Radikal aus der Gruppe von Phenyl, Naphthyl oder Phenanthryl, wobei ein solches aromatisches Radikal $R^6$ oder $R^7$ unsubstituiert oder durch einen oder mehrere Reste $Z^5$ bzw. $Z^7$ substituiert sein kann,
$Z^6$ Phenyl-nieder-alkoxy, Hydroxyimino-nieder-alkyl oder eine Gruppe Z,
$Z^7$ Phenyl-$C_{2-7}$-alkoxy oder eine Gruppe Z,
Z Cl, F, nieder-Alkyl, nieder-Alkoxy, nieder-Alkanoyl, nieder-Alkanoyloxy, Hydroxy-nieder-alkyl, COOH, nieder-Alkoxycarbonyl, $NH_2$, Amino-nieder-alkyl, Mono- oder Di-nieder-alkylamino, Mono- oder Di-nieder-alkylamino-nieder-alkyl, nieder-Alkanoylamino, $CONH_2$, Mono- oder Di-nieder-alkylcarbamoyl, Trifluoracetylamino, $CF_3$, OH oder Pyridyl ist; oder Z mit zwei benachbarten C-Atomen die Gruppierung -$OCH_2CH_2N$-($R''$)- oder -$OCH_2CON$($R'''$)- bildet,
$R''$ H, nieder Alkanoyl oder Trifluoracetyl,
$R'''$ H oder nieder Alkyl,
$R^8$, $R^9$ und $R^{10}$ H, OH, nieder Alkyl, nieder Alkoxy, Hydroxy-nieder-alkyl, F, Cl oder nieder-Alkanoyl sind, jedoch nicht mehr als eins von $R^8$, $R^9$ oder $R^{10}$ OH, nieder-Alkoxy, Hydroxy-nieder-alkyl, F, Cl oder nieder-Alkanoyl ist, und
Y CH oder N ist,
und Salze davon.

2. Verbindungen gemäss Anspruch 1, worin A die gleiche Bedeutung wie in Anspruch 1 hat, mit der Ausnahme, dass $R^6$ und $R^7$ nicht durch nieder-Alkanoyloxy substituiert ist und $R^7$ nicht durch Amino-nieder-alkyl, Mono- oder Di-nieder-alkylamino oder Mono- oder Di-nieder-alkylamino-nieder-alkyl substituiert ist.

3. Verbindungen gemäss Anspruch 1 oder 2, worin $R^1$ H und $R^2$ bis $R^5$ Methyl und A -C≡C-$R^6$ sind.

4. Verbindungen gemäss Anspruch 1, 2 oder 3, worin $R^6$ Thienyl, Pyridyl, Benzothienyl oder substituiertes oder unsubstituiertes Phenyl ist.

5. Verbindung nach Anspruch 1 oder 2, worin $R^1$ H, $R^2$ bis $R^5$ Methyl und A A' ist, worin Y N und $R^8$

bis $R^{10}$ H oder nieder-Alkyl sind.

6. [2-(Benzo[b]thien-3-yl)äthinyl]-3,4-dihydro-2,5,7,8-tetramethyl-2H-1-benzopyran-6-ol in S(+)-, RS- oder, insbesondere in R(-)-Form.

7. Eine Verbindung gemäss Anspruch 1 oder 2, aus der Gruppe der folgenden:
3,4-Dihydro-2,5,7,8-tetramethyl-2-[(2-thienyl)äthinyl]-2H-1-benzopyran-6-ol in R-, S- oder RS-Form,
3,4-Dihydro-2-(furo[3,2-b]pyridin-2-yl)-2,5,7,8-tetramethyl-2H-1-benzopyran-6-ol,
2-(2-Benzofuranyl)-3,4-dihydro-2,5,7,8-tetramethyl-2H-1-benzopyran-6-ol und
2-(3,4-Dihydro-6-hydroxy-2,5,7,8-tetramethyl-2H-1-benzopyran-2-yl)-7-methoxybenzofuran-5-methanol.

8. Die Verbindungen nach einem der Ansprüche 1 bis 7 zur Verwendung als Medikamente.

9. Verfahren zur Herstellung der Verbindungen gemäss Anspruch 1, dadurch gekennzeichnet, dass man
a) eine Verbindung der Formel

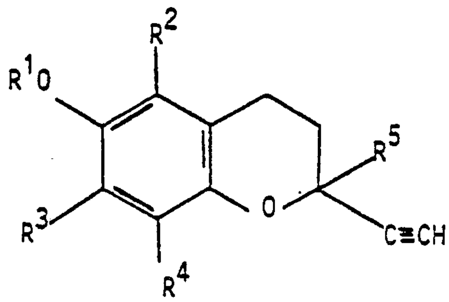

II

mit einer Verbindung der Formel $R^6X$, worin X Br, I oder Trifluormethylsulfonyloxy sind und $R^1$-$R^6$ die obige Bedeutung haben, umsetzt oder

b) eine Verbindung der Formel I, worin A -C≡C-$R^6$ ist, hydriert,

c) eine durch die Reaktion a) entstandene Verbindung der Formel I, worin A -C≡C-$R^{61}$ und $R^{61}$ die Gruppe der Formel

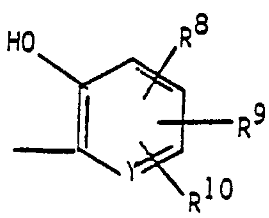

$R^{61}$

ist, in dieser Form oder in Form der entsprechenden Verbindung der Formel I, worin A die Gruppe A′ ist, isoliert,

d) gewünschtenfalls eine nach Reaktion a), b) oder c) entstandene Verbindung der Formel I in ein Salz überführt.

10. Pharmazeutische Präparate auf der Basis einer Verbindung gemäss einem der Ansprüche 1 bis 7.

11. Verwendung der Verbindungen gemäss einem der Ansprüche 1 bis 7 bei der Herstellung eines pharmazeutischen Präparats zur Hemmung der 5-Lipoxygenase und der Lipidperoxidation.

Patentansprüche für folgende Vertragsstaaten: GR, ES

1. Verfahren zur Herstellung von Benzopyranolderivaten der Formel

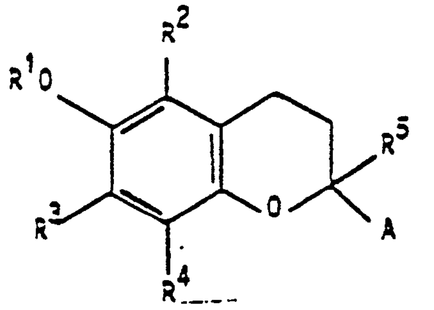

I

worin A $-C{\equiv}C-R^6$, $-CH_2CH_2-R^7$ oder

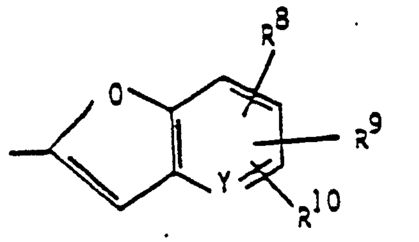

A'

und $R^1$ Wasserstoff oder nieder-Alkanoyl,
$R^2$, $R^3$ und $R^4$ Wasserstoff oder nieder-Alkyl,
$R^5$ nieder-Alkyl,
$R^6$ und $R^7$ ein heteroaromatisches Radikal oder ein aromatisches Radikal aus der Gruppe von Phenyl, Naphthyl oder Phenanthryl, wobei ein solches aromatisches Radikal $R^6$ oder $R^7$ unsubstituiert oder durch einen oder mehrere Reste $Z^6$ bzw. $Z^7$ substituiert sein kann,
$Z^6$ Phenyl-nieder-alkoxy, Hydroxyimino-nieder-alkyl oder eine Gruppe Z,
$Z^7$ Phenyl-$C_{2-7}$-alkoxy oder eine Gruppe Z,
Z Cl, F, nieder-Alkyl, nieder-Alkoxy, nieder-Alkanoyl, nieder-Alkanoyloxy, Hydroxy-nieder-alkyl, COOH, nieder-Alkoxycarbonyl, $NH_2$, Amino-nieder-alkyl, Mono- oder Di-nieder-alkylamino, Mono- oder Di-nieder-alkylamino-nieder-alkyl, nieder-Alkanoylamino, $CONH_2$, Mono- oder Di-nieder-alkylcarbamoyl, Trifluoracetylamino, $CF_3$, OH oder Pyridyl ist; oder Z mit zwei benachbarten C-Atomen die Gruppierung $-OCH_2CH_2N(R'')-$ oder $-OCH_2CON(R''')-$ bildet,
$R''$ H, nieder Alkanoyl oder Trifluoracetyl,
$R'''$ H oder nieder Alkyl,
$R^8$, $R^9$ und $R^{10}$ H, OH, nieder Alkyl, nieder Alkoxy, Hydroxy-nieder-alkyl, F, Cl oder nieder-Alkanoyl sind, jedoch nicht mehr als eins von $R^8$, $R^9$ oder $R^{10}$ OH, nieder-Alkoxy, Hydroxy-nieder-alkyl, F, Cl oder nieder-Alkanoyl ist, und
Y CH oder N ist,
und Salze davon, dadurch gekennzeichnet, dass man

a) eine Verbindung der Formel

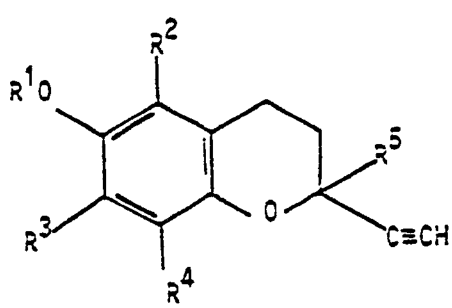

II

mit einer Verbindung der Formel $R^6X$, worin X Br, I oder Trifluormethylsulfonyloxy sind und $R^1$-$R^6$ die obige Bedeutung haben, umsetzt oder

b) eine Verbindung der Formel I, worin A -C≡C-$R^6$ ist, hydriert,

c) eine durch die Reaktion a) entstandene Verbindung der Formel I, worin A -C≡C-$R^{61}$ und $R^{61}$ die Gruppe der Formel

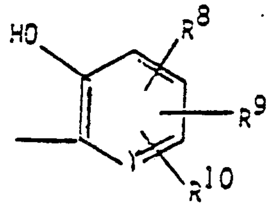

ist, in dieser Form oder in Form der entsprechenden Verbindung der Formel I, worin A die Gruppe A′ ist, isoliert,

d) gewünschtenfalls eine nach Reaktion a), b) oder c) entstandene Verbindung der Formel I in ein Salz überführt.

2. Verfahren gemäss Anspruch 1, worin A die gleiche Bedeutung wie in Anspruch 1 hat, mit der Ausnahme, dass $R^6$ und $R^7$ nicht durch nieder-Alkanoyloxy substituiert ist und $R^7$ nicht durch Amino-nieder-alkyl, Mono- oder Di-nieder-alkylamino oder Mono- oder Di-nieder-alkylamino-nieder-alkyl substituiert ist.

3. Verfahren gemäss Anspruch 1 oder 2, worin $R^1$ H und $R^2$ bis $R^5$ Methyl und A -C≡C-$R^6$ sind.

4. Verfahren gemäss Anspruch 1, 2 oder 3, worin $R^6$ Thienyl, Pyridyl, Benzothienyl oder substituiertes oder unsubstituiertes Phenyl ist.

5. Verfahren nach Anspruch 1 oder 2, worin $R^1$ H, $R^2$ bis $R^5$ Methyl und A A′ ist, worin Y N und $R^8$ bis $R^{10}$ H oder nieder-Alkyl sind.

6. Verfahren nach Anspruch 1, dadurch gekennzeichnet, dass man das [2-(Benzo[b]thien-3-yl)äthinyl]-3,4-dihydro-2,5,7,8-tetramethyl-2H-1-benzopyran-6-ol in S(+)-, RS- oder, insbesondere in R(-)-Form herstellt.

7. Verfahren nach Anspruch 1, dadurch gekennzeichnet, dass man eine Verbindung gemäss Anspruch 1 oder 2, aus der Gruppe der folgenden herstellt:
3,4-Dihydro-2,5,7,8-tetramethyl-2-[(2-thienyl)äthinyl]-2H-1-benzopyran-6-ol in R-, S- oder RS-Form,
3,4-Dihydro-2-(furo[3,2-b]pyridin-2-yl)-2,5,7,8-tetramethyl-2H-1-benzopyran-6-ol,
2-(2-Benzofuranyl)-3,4-dihydro-2,5,7,8-tetramethyl-2H- 1-benzopyran-6-ol und
2-(3,4-Dihydro-6-hydroxy-2,5,7,8-tetramethyl-2H-1-benzopyran-2-yl)-7-methoxybenzofuran-5-methanol.

8. Verfahren nach Anspruch 1, dadurch gekennzeichnet, dass man

a) eine Verbindung der Formel II mit einer solchen der Formel $R^6X$ in Gegenwart eines Palladiumkatalysators und eines Protonenakzeptors in einem inerten organischen Lösungsmittel bei einer Temperatur bis zu 100 °C umsetzt oder

b) eine Verbindung der Formel I, worin A -C≡C-$R^6$ ist, mittels eines Edelmetallkatalysators, in Gegenwart eines inerten Lösungsmittels bei einer Temperatur bis zu 50 °C unter einem Druck von 1-20 Atm. hydriert.

9. Verfahren zur Herstellung eines pharmazeutischen Präparates, dadurch gekennzeichnet, dass man eine Verbindung der Formel I oder ein Salz davon in eine galenische Verabreichungsform bringt.

10. Verwendung der Verbindungen gemäss einem der Ansprüche 1 bis 7 bei der Herstellung eines pharmazeutischen Präparats zur Hemmung der 5-Lipoxygenase und der Lipidperoxidation.